## Europäisches Patentamt
## European Patent Office
## Office européen des brevets

(19)

(11) Numéro de publication: **0 101 341**
**B1**

(12) # FASCICULE DE BREVET EUROPÉEN

(45) Date de publication du fascicule du brevet:
**05.11.86**

(51) Int. Cl.⁴: **A 61 K 9/50**, A 61 K 35/18, A 61 M 1/14, B 01 D 13/00

(21) Numéro de dépôt: **83401364.1**

(22) Date de dépôt: **01.07.83**

(54) **Procédé et dispositif pour l'encapsulation dans les érythrocytes d'au moins une substance à activité biologique, notamment des effecteurs allostériques de l'hémoglobine et érythrocytes ainsi obtenus.**

(30) Priorité: **05.07.82 FR 8211749**

(43) Date de publication de la demande:
**22.02.84 Bulletin 84/8**

(45) Mention de la délivrance du brevet:
**05.11.86 Bulletin 86/45**

(84) Etats contractants désignés:
**AT BE CH DE FR GB IT LI LU NL SE**

(56) Documents cité:
FR-A-2 259 904
FR-A-2 373 289
FR-A-2 373 290
FR-A-2 374 045

BIOLOGICAL ABSTRACTS, vol. 71, 1981, abrégé 75061, Philadelphia, US W.E. LYNCH et al.: "Erythrocytes as carriers of chemotherapeutic agents for targeting the RES"
BIOLOGICAL ABSTRACTS, vol. 70, 1980, abrégé 73456, Philadelphia, US M.B. FIDDLER et al.: "Enzyme therapy: 14. Comparison of methods for enzyme entrapment in human erythrocytes"
CHEMICAL ABSTRACTS, vol. 93, no. 2, 14 juillet 1980, page 308, no. 12977g, Columbus, Ohio, US T. KITAO et al.: "Co-entrapment of glucose oxidase and catalase into erythrocytes"
BIOLOGICAL ABSTRACTS, vol. 71, 1981, abrégé 40509, Philadelphia, US J.D. HUMPHREYS et al.: "Enhanced stability of erythrocyte entrapped glucocerebrosidase activity"
CHEMICAL ABSTRACTS, vol. 87, no. 26, 26 décembre 1977, page 286, no. 206475p, Columbus, Ohio, US G. SCHMER et al.: "Gel-bound resealed

(73) Titulaire: **Etablissement Public dit: CENTRE NATIONAL DE LA RECHERCHE SCIENTIFIQUE (CNRS), 15, Quai Anatole France, F-75700 Paris (FR)**
Titulaire: **CENTRE HOSPITALIER REGIONAL DE TOURS, 2 boulevard Tonnellé, F-37000 Tours (FR)**
Titulaire: **Studiengesellschaft Kohle mbH, Kaiser-Wilhelm- Platz 1, D-4330 Mülheim/Ruhr (DE)**

(72) Inventeur: **Ropars, Claude, rue Toulouse Lautrec, F-37000 Tours (FR)**
Inventeur: **Nicolau, Yves Claude, 1 rue de l'Ardoise, F-45380 La Chapelle St Mesmin (FR)**
Inventeur: **Chassaigne, Maurice, 26, rue de la Choizille, F-37100 St Cyr Sur Loire (FR)**

(74) Mandataire: **Warcoin, Jacques, Cabinet Régimbeau 26, avenue Kléber, F-75116 Paris (FR)**

(56) Documents cité: (suite)
red cell membranes: a new type of semi-artificial organ"
CHEMICAL ABSTRACTS, vol. 86, no. 11, 14 mars 1977, page 17, no. 65360n, Columbus, Ohio, US D.A. TYRRELL et al.: "The entrapment of therapeutic agents in resealed erythrocyte ghosts and their fate in vivo"
CHEMICAL ABSTRACTS, vol. 88, no. 6, 6 février 1978, page 248, no. 41621p, Columbus, Ohio, US S. UPDIKE: "Strategy for enzyme therapy: immobilization in hypoallergenic gel versus entrapment in red blood cells"
Journal Lab. Clin. Med. August 1980, pp. 307-317

**0 101 341**

## Description

La présente invention concerne un procédé d'encapsulation dans les érythrocytes humains ou animaux d'au moins une substance présentant une activité biologique, un dispositif utile pour la mise en oeuvre de ce procédé et les érythrocytes obtenus.

Préalablement à l'exposé de la présente invention, il peut être intéressant de rappeler certaines notions en la matière.

Le sang est un mélange complexe constitué d'un liquide, le plasma, dans lequel sont en suspension des globules rouges (érythrocytes) transportant l'oxygène, des globules blancs (leucocytes) et les plaquettes. Le plasma contient en solution des sels, des protéines et des métabolites solubles qui s'échangent dans les différents tissus.

Dans le cadre de la présente invention, seuls les érythrocytes nous intéressent.

De façon simplifiée, on peut considérer que les érythrocytes sont constitués d'une membrane qui entoure un cytoplasme rempli essentiellement d'hémoglobine.

Le rôle de l'hémoglobine est de se combiner à l'oxygène moléculaire pendant le passage des érythrocytes à travers les poumons et de transporter cet oxygène à tous les tissus du corps pour qu'il y soit utilisé.

Comme pour toutes les cellules, les érythrocytes peuvent être lysés dans certaines conditions, en particulier de pression osmotique, mais c'est en 1952 que Teorrel a introduit la notion de rescellement de la membrane érythrocytaire, c'est-à-dire de la reconstitution de la membrane érythrocytaire après une lyse des érythrocytes.

Dans la mesure où il devenait ainsi possible d'ouvrir et de refermer les érythrocytes, il devait donc être possible d'y introduire certains composants exogènes. Outre la technique de base qui consiste à mettre les érythrocytes dans une solution contenue dans un récipient dont on fait successivement changer la pression osmotique afin d'effectuer la lyse puis le rescellement, il faut citer certains procédés mettant en oeuvre une lyse en milieu iso-osmotique en appliquant aux érythrocytes un choc électrique ou thermique. Dans certains cas il a également été utilisé des virus ou des protéines virales afin de réaliser cette encapsulation.

Il a également été réalisé des encapsulations par dialyse dans un sac de cellophane. Tous les procédés utilisés précédemment ont en commun de présenter des rendements d'encapsulation faibles, d'être excessivement aléatoires quant à leur reproductibilité, et de ne permettre de traiter que des quantités faibles de suspensions érythrocytaires.

Ainsi, bien que l'on ait rapporté l'encapsulation de certaines enzymes du facteur IX de la desferrioxamine, des acides organiques, du glucose, du saccharose, du fragment A de l'anti-toxine diphtérique et du méthothrexate, ces procédés sont restés au stade du laboratoire et n'ont pas permis jusqu'à présent leur utilisation chez l'homme.

Seule une publication récente de Green R. et al. (The Lancet (1980), 16 août, 327-330) décrit l'encapsulation de Desféral ® (commercialisé par CIBA-GEIGY) et l'utilisation des érythrocytes obtenus chez l'homme.

Cette encapsulation ayant été réalisée par simple dilution en milieu hypo-osmotique montre toutefois des rendements très faibles d'incorporation du Desféral dans les érythrocytes.

C'est pourquoi la présente invention a pour but la mise au point d'un procédé et d'un dispositif permettant de réaliser l'encapsulation dans les érythrocytes de substances présentant une activité biologique avec un rendement d'encapsulation très élevé et permettant de traiter des grandes quantités de suspensions d'érythrocytes tout en assurant le maintien de bonnes conditions de stérilité qui permettent d'utiliser les érythrocytes ainsi obtenus chez l'homme ou l'animal.

Bien que la présente invention ait été développée pour l'encapsulation d'un grand nombre de composés à activité biologique, l'une des classes de composés à activité biologique paraît plus partie, il s'agit des effecteurs allostériques de l'hémoglobine.

L'hémoglobine forme avec l'oxygène au niveau des poumons un produit d'addition réversible l'oxyhémoglobine.

Ce produit d'addition se décompose au niveau du système capillaire pour libérer l'oxygène car la pression partielle de l'oxygène dans le système capillaire est plus faible (de l'ordre de $100.10^2Pa$ -70 mm de Hg) que dans les poumons (de l'ordre de $133.10^2Pa$ - 100 mm de Hg)

Dans les conditions normales, seule une partie, environ 25 %, de l'hémoglobine se dissocie, le reste retourne aux poumons par le système veineux.

In vitro, il a été possible de montrer le rôle de certaines substances comme l'inositol hexaphosphate (IHP), l'inositol pentaphosphate (IPP), le pyridoxal phosphate, par exemple, qui sont susceptibles de se combiner avec l'hémoglobine au niveau du site de fixation du 2,3-diphosphoglycérate.

Ces produits dénommés "effecteurs allostériques de l'hémeglobine" (EAH) modifient la conformation allostérique de l'hémoglobine, ce qui a pour effet de diminuer l'affinité de l'hémoglobine pour l'oxygène.

La diminution de l'affinité de l'hémoglobine pour l'oxygène rend plus facile la libération de l'oxygène à partir de l'oxyhémoglobine, même pour des pressions partielles d'oxygène relativement élevées.

Dans ces conditions, sans que le contenu global en oxygène du sang soit notablement modifié, il est possible d'envisager d'accroître le pouvoir oxyphorique du sang vis à vis des tissus en utilisant des globules rouges comportant une hémoglobine modifiée.

Ce type de traitement de l'hémoglobine permet une meilleure oxygénation du tissu par amélioration de la libération de l'oxygène.

On conçoit que les applications de ces effecteurs allostériques de l'hémoglobine puissent être nombreuses,

2

par exemple dans le traitement de certaines affections telles que les insuffisances respiratoires, les troublatoires, ou bien pour améliorer ou accélérer certains processus thérapeutiques, par exemple dans la prévention de l'infarctus du myocarde lié à certaines ischémies, ou pour modifier la physiologie respiratoire à de grandes altitudes ou profondeurs.

Le problème essentiel est, bien entendu, d'amener les effecteurs in situ afin qu'ils puissent se fixer sur l'hémoglobine intracellulaire.

Les tentatives de transformation de l'hémoglobine en solution par les effecteurs allostériques de l'hémoglobine ont pu être réalisées in vitro, mais les applications pratiques ne sont pas encore susceptibles d'une utilisation thérapeutique du fait de la durée de vie insuffisante de l'hémoglobine en solution et des propriétés réduites qu'elle a alors dans le transport de l'oxygène.

Le brevet des Etats-Unis d'Amérique n° 4 192 869 et le brevet européen n° 0 001 104 décrivent des procédés destinés à transformer les globules rouges par interaction desdits globules avec des liposomes chargés en effecteurs allostériques de l'hémoglobine, en particulier chargés en IHP.

Le procédé et le dispositif selon la présente invention permettent en utilisant comme composé à activité biologique un effecteur allostérique de l'hémoglobine, la transformation de l'hémoglobine dans des conditions qui autorisent son utilisation au niveau thérapeutique.

Pour ce faire, la présente invention propose un procédé d'encapsulation dans les érythrocytes humains ou animaux d'au moins une substance présentant une activité biologique, caractérisé en ce qu'on alimente en continu, avec une suspension aqueuse d'érythrocytes, le compartiment primaire d'au moins un élément de dialyse dont le compartiment secondaire contient une solution aqueuse hypotonique par rapport à la dite suspension aquense d'érythrocytes afin de lyser les érythrocytes, en ce que le lysat d'érythrocytes est alors mis en contact avec ladite substance présentant une activité biologique, et en ce que ensuite, afin de resceller la membrane des érythrocytes, on augmente la pression osmotique et/ou oncotique du lysat érythrocytaire. Généralement on recueille alors la suspension d'érythrocytes rescellés.

Dans le cadre de la présente description, on entendra par "élément de dialyse" de façon générale un élément comportant deux compartiments séparés par une paroi de dialyse à travers laquelle peut se faire un échange ionique qui permet de modifier de façon contrôlée la pression osmotique d'une solution aqueuse située dans l'un des compartiments en introduisant dans l'autre compartiment une solution aqueuse d'un sel. Ce type d'élément de dialyse est largement utilisé tant dans le domaine médical pour la mise en oeuvre d'hémodialyse en flux continu, par exemple de dialyse péritonéale ou d'échange plasmatique avec séparation des cellules et du plasma, que dans le domaine industriel dans des opérations de purification, par exemple dans l'industrie pharmaceutique et alimentaire.

Il doit être bien entendu que l'invention ne porte pas sur la structure d'un tel élément de dialyse qui peut être quelconque et être constitué, par exemple, par des membranes ou des fibres creuses avec des cheminements quelconques à travers plusieurs chambres de dialyse, l'aspect essentiel du procédé étant que l'on puisse modifier la tonicité de la solution aqueuse introduite dans le compartiment primaire grâce à l'adjonction dans le compartiment secondaire d'une solution aqueuse hypotonique par rapport à la suspension d'érythrocytes, ceci bien entendu afin de lyser les érythrocytes.

Le lysat d'érythrocytes doit être en contact avec la substance présentant une activité biologique que l'on souhaite encapsuler, mais il est possible d'introduire cette substance soit avant, soit pendant la lyse, soit même éventuellement après la lyse, toutefois, dans ce dernier cas1 biologique, deux situations peuvent se présenter:

a) La taille de la substance à encapsuler est supérieure à la taille des pores de la membrane qui sépare compartiments primaire et secondaire du dialyseur. Ce sera le cas d'une substance macromoléculaire. Dans ces conditions, il n'y aura aucune perte de cette substance au cours de la dialyse conduisant à la phase d'introduction de la substance dans les hématies, le rendement final correspondra à la phase d'équilibre obtenu après la lyse des hématies.

b) La substance ü encapsuler a une taille réduite (inférieure à 10 000 daltons), elle dialysera donc dans le compartiment secondaire du dispositif au cours de la phase de lyse. Ce qui conduira à une diminution du rendement d'encapsulation de la substance dans les hématies. Pour réduire cette perte de substance qui peut être très préjudiciable dans le cas de composés coûteux ou rares, il est possible de modifier le procédé de la façon qui suit:

En effet, les hématies sont initialement lavées dans un milieu contenant des solutés dont la pression osmotique est d'environ 220 à 300 mos. La lyse de ces hématies ne se produit normalement qu'en dessous de 200 mos. C'est au cours de la phase d'abaissement de la pression osmotique jusqu'à 200 mos que se produit la perte principale de la substance à encapsuler, si elle a été introduite préalablement au début de l'opération de dialyse. Pour obtenir une réduction de cette perte par diffusion, il suffit donc de réaliser l'introduction de la substance dans le milieu au moment où commence la lyse des hématies, c'est-àdire lorsque la tonicité de la suspension a été amenée entre 180 et 220 mos.

Dans cette variante, avant d'introduire la suspension d'érythrocytes dans le premier élément de dialyse, on abaisse la pression osmotique de la suspension jusqu'à 180-220 mos et c'est seulement lorsque la pression osmotique atteint cette valeur que l'on injecte dans la suspension la substance à encapsuler. La suspension ainsi obtenue est alors soumise aux différentes étapes décrites précédemment.

L'abaissement de la tonicité à 180-220 mos est réalisé de préférence en utilisant un élément de dialyse supplémentaire placé en amont des éléments déjà décrits. Dans cet élément de dialyse, on fait circuler la suspension érythrocytaire dans l'un des compartiments et une solution hypotonique dans l'autre, ceci a pour effet d'abaisser la tonicité de la suspension d'érythrocytes.

Dans ce mode de réalisation, la substance à encapsuler est introduite dans l'effluent de l'élément de dialyse supplémentaire et ensuite la suspension ainsi traitée est introduite dans l'élément de dialyse dont les caractéristiques sont choisies de telle sorte que la lyse soit optimale à la sortie de cet élément.

Il existe plusieurs possibilités afin d'augmenter la pression osmotique du lysat d'érythrocytes lorsque l'on désire resceller la membrane des érythrocytes.

Dans un premier mode de réalisation, on augmente la pression osmotique du lysat d'érythrocytes en le faisant passer dans le circuit primaire d'un élément de dialyse dont le circuit secondaire contient une solution hypertonique par rapport au lysat, la solution après rescellement étant recueillie en continu.

Dans un second mode de réalisation, on augmente la pression osmotique du lysat en le mélangeant avec une solution hypertonique et/ou hyper-oncotique par rapport audit lysat.

Dans ce qui précède, il convient de remarquer que le produit traité est une suspenion aqueuse d'érythrocytes, c'est-à-dire que dans la plupart des cas on préférera travailler sur une suspension "synthétique" d'érythrocytes et non sur le sang total, c'est-à-dire une suspension ne comportant principalement comme composant d'origine sanguine que les érythrocytes.

C'est pourquoi, outre les phases de dialyse-rescellement , le procédé peut faire intervenir d'une manière prépondérante des phases de:
- lavages et préparation des hématies, avant lyse et rescellement,
- lavages et éventuellement remise en suspension plasmatique ou de composition artificielle des hématies transformées.

De nombreux procédés et variantes actuellement connus peuvent être ajoutés aux dispositifs permettant l'encapsulation de substance dans les globules rouges selon le procédé. Il s'agit essentiellement des méthodes de:
- centrifugation pour la séparation cellules-plasma et pour les lavages,
- séparation à l'aide de séparateurs de cellules en flux continu ou semi-continu,
- utilisation de dispositifs en flux continu et semi-continu pour la séparation des cellules sur membranes semi-perméables selon les méthodes actuelles de plasmaphorèse sur membrane ou sur fibres creuses,
- lavages selon les mêmes procédés.

En outre, il est bien connu en transfusion sanguine, qqu'il est parfois souhaitable d'éliminer les globules blancs ou les plaquettes avant transfusion pour des raisons soit immunologiques soit liées à la formation de microaggrégats.

Les mêmes situations peuvent se rencontrer pour l'utilisation de globules rouges lysés et rescellés ayant encapsulé une substance d'intérêt biologique. Il peut donc être envisagé l'introduction dans le procédé d'un dispositif de déleucocytation tel qu'un filtre à absorption conventionnel. Les autres procédés classiques de déleucocytation peuvent évidemment être adaptés au dispositif.

L'utilisation d'une suspension "synthétique" d'érythrocytes permet de contrôler avec précision la pression osmotique de ladite suspension aqueuse et donc assure une bonne reproductibilité des résultats obtenus.

Lorsque l'on prépare cette suspension aqueuse d'érythrocytes, il est possible d'effectuer divers types de "conditionnement" de la suspension.

On peut tout d'abord placer les érythrocytes dans un milieu iso-osmotique par rapport au sang, c'est-à-dire dans les conditions naturelles de pression osmotique, dans ces conditions la lyse sera effectuée en présence d'une solution aqueuse hypotonique par rapport à la suspension d'érythrocytes.

Mais il est également possible de conditionner les érythrocytes dans une solution aqueuse contenant des substances diffusibles à travers la membrane, dans ces conditions ces érythrocytes vont avoir tendance à gonfler et il sera possible d'effectuer la lyse en utilisant un milieu iso-osmotique ou même, dans certains cas, hyper-osmotique (ces pressions étant évaluées par rapport à la pression osmotique normale dans le sang).

Il peut être également intéressant lorsque l'on prépare la suspension synthétique d'érythrocytes destinée à être traitée d'ajouter à cette suspension des composés macromoléculaires, en particulier des colloïdes, de façon à créer dans cette solution une pression oncotique, cette pression oncotique aura pour effet lors de la lyse de limiter la perte des macromolécules intracytoplasmiques et en particulier la perte de l'hémoglobine.

Il est bien entendu possible d'envisager, au stade de la lyse ou du rescellement, d'utiliser non seulement des modifications de la pression osmotique de la solution, mais également des modifications de la pression oncotique afin d'améliorer la lyse ou le rescellement.

L'un des avantages considérable du procédé selon la présente invention, outre les possibilités de contrôler la stérilité et la présence de pyrogènes,ce qui permet l'utilisation des produits obtenus chez l'homme, est que ce procédé se prête particulièrement bien à un asservissement par contrôle des paramètres tels que temps, volume, température, débit, surface d'échange, conductivité, force ionique, notamment.

Les différents paramètres de mise en oeuvre du procédé peuvent varier suivant la nature des éléments traités mais la lyse sera effectuée, de préférence, à une température comprise entre 0 et 10°C et lé rescellement à une température comprise entre 20 et 40°C.

L'analyse du phénomène de rescellement des hématies permet de considérer comme possible l'intervention de conditions différentes de celles qui ont été décrites ci-dessus:
- introduction de la substance à encapsuler à 2-4°C dans la suspension d'hématies lysées et non avant l'entrée dans le circuit de dialyse;

- étapes à des températures différentes, par palliers successifs, conduisant généralement à un passage de la température de 2 à 6°C au départ à 35 - 42°C entre la lyse et le rescellement;
- introduction de solutions ou substances de rescellement à différents points des palliers précédents;
- compositions variables des solutions ou tampons de rescellement.

Enfin, l'encapsulation de substances très actives à des concentrations faibles qui possèdent un caractère protéique ou facilement adsorbables sur les surfaces plastiques utilisées dans le procédé conduit à envisager dans certains cas un prétraitement du circuit de dialyse. Ces phénomènes d'absorption sont bien connus et peuvent conduire, compte tenu des grandes surfaces utilisées, à la perte dans le procédé d'une quantité trés importante de la substance à encapsuler. Ceci pourraît être par exemple le cas de substances comme les cytokines ou les enzymes. Diverses méthodes peuvent être utilisées pour modifier les surfaces des circuits de transformation, comme par exemple l'utilisation préalable d'un recouvrement par une protéine comme l'albumine.

Ces précautions méthodologiques ne sont pas directement liées au procédé mais peuvent jouer un rôle majeur dans son utilisation.

Comme cela a été indiqué précédemment, il a été constaté que bien que le procédé selon la présente invention soit un procédé en continu, on obtenait des rendements très supérieurs aux rendements observés par la technique dite en "sac de dialyse". En outre, les volumes de sang traités peuvent être très importants, le traitement s'effectuant avec une grande rapidité.

Les différences observées entre ces résultats peuvent s'expliquer en partie car les deux procédés sur le plan fondamental sont très différents. En effet, l'un des procédés est une méthode cinétique alors que l'autre est une méthode d'équilibre. Dans ces conditions, le raccourcissement considérable de la durée durant laquelle les hématies sont maintenues lysées limite les échanges physicochimiques membrane-milieu s'accompagnant d'une perte de constituants de la membrane érythrocytaire, ce qui réduit fortement la proportion d'hématies pour lesquelles la lyse est devenue irréversible.

Bien entendu, outre les avantages liés à la rapidité de traitement qui peut être obtenue par la mise en oeuvre du procédé, il faut mentionner le fait qu'il est possible, pour la phase de dialyse, de traiter des volumes importants, par exemple il est possible de traiter 200 ml de culot globulaire lavé en 10 minutes au lieu de 10 à 20 ml en 2 heures et demie par la méthode en sac de dialyse.

Le procédé selon la présente invention peut être mis en oeuvre pour encapsuler un grand nombre de substance présentant une activité biologique. Il s'agit en particulier:
- des protéines,
- des enzymes,
- des hormones,
- des substances à activité pharmacologique de façon générale,
- des substances modifiant le métabolisme des érythrocytes, telles que, par exemple, les effecteurs allostériques de l'hémoglobine, et
- des substances protectrices, notamment cryoprotectrices, de l'hémoglobine et des érythrocytes.
- les acides nucléiques.

Comme cela a été développé précédemment, parmi les substances présentant une activité biologique qui peuvent être mises en oeuvre dans le procédé de l'invention, il faut citer les effecteurs allostériques de l'hémoglobine.

Parmi les effecteurs allostériques de l'hémoglobine, il faut mentionner l'inositol hexaphosphate (IHP), mais d'autres effecteurs sont utilisables, par exemple les sucres phosphates tels que l'inositol pentaphosphate, l'inositol tétraphosphate, l'inositol triphosphate, l'inositol diphosphate et le diphosphatidynilinositol diphosphate.

Il est possible d'utiliser également des polyphosphates tels que des nucléotides triphosphates, nucléotides diphosphates, nucléotides monophosphates, des esters phosphate-alcools et le phosphate de pyridoxal.

Toute une série de substances naturelles ou synthétiques présentant un intérêt croissant en thérapeutique cellulaire ou moléculaire peuvent être encapsulées:
- les prostaglandines
- les leukotriènes
- les cytokines ou immunomodulateurs de synthèse (immunoactivateurs ou immunosuppresseurs); dans cette dernière catégorie, il peut être utile d'envisager d'utiliser le procédé pour moduler le système de défense immunitaire de l'organisme à l'aide de substances naturelles ou synthétiques que le procédé permet de cibler:
- sur le système réticuloendothélial, lieu privilégié de disparition des érythrocytes au cours du vieillissement;
- sur certaines cellules comme les monocytes, destinés à se transformer en macrophages tissulaires, en particulier à l'aide d'une réaction de cytolyse anticorps dépendante (ADCC);
- sur le système lymphatique qui représente le lieu privilégié d'élimination d'érythrocytes transfusés, par exemple par voie intrapéritonéale.

Les composés les plus intéressants sont actuellement:
- les interférons $\alpha$, $\beta$ ou $\gamma$ ou de recombinaison génétique;
- l'interleukine II;
- les immunomodulateurs de synthèse, en particulier les dérivés du muramyldipeptide (MDP).

Pour les mêmes raisons qui permettent d'envisager le ciblage sélectif et efficace des immunomodulateurs naturels ou synthétiques, outre un effet retard et la limitation de la toxicité, il peut être très utile et efficace d'utiliser le procédé pour l'encapsulation, le ciblage et la mise en action de substances douées de propriétés anticancéreuses.

Parmi les nombreuses autres substances naturelles, en particulier les enzymes, que le procédé permet d'encapsuler, il est possible de donner de nombreux exemples.

Il faut en mentionner trois plus particulièrement intéressantes:

a) Encapsulation d'enzymes du métabolisme du glucose Le glucose est un substrat qui diffuse rapidement et librement à travers la membrane du globule rouge.

L'encapsulation d'enzymes du métabolisme du glucose, particulièrement des hexokinases, pourrait permettre d'agir sur le métabolisme extraérythrocytaire du glucose, et donc de moduler la concentration sanguine du glucose, en dehors de l'action d'hormones comme l'insuline.

La stabilité intraérythrocytaire pourrait éventuellement nécessiter l'addition d'inhibiteurs de protéases comme dans le cas de l'insuline ou d'autres inhibiteurs enzymatiques.

b) Encapsulation d'anhydrase carbonique

Le métabolisme du $CO_2$ pose de graves problèmes dans la réanimation de certains malades présentant une forte surcharge, mal maîtrisée jusqu'à présent, du $CO_2$ sanguin.

L'encapsulation d'anhydrase carbonique, en plus des concentrations naturellement importantes dans le globule rouge, pourrait permettre de déplacer l'équilibre: $CO_2 \rightarrow CO_3H^-$ ce dernier composé pouvant être éliminé par dialyse du sang du malade.

c) Encapsulation d'enzymes du métabolisme de l'histamine

Il a été vérifié que l'histamine diffuse à travers la membrane érythrocytaire d'une manière analogue à celle du glucose, mais avec une cinétique plus lente.

Ce point fondamental permet d'utiliser le procédé pour l'encapsulation d'enzymes du métabolisme de l'histamine, en particulier: la diamino-oxidase (histaminase) et la méthylhistamine transférase, qui sont les deux enzymes privilégiées du catabolisme de l'histamine.

Ce point permet d'étudier une éventuelle adaptation du procédé pour le traitement de surcharges en histamine, en particulier chroniques, chez les sujets allergiques.

Ces exemples illustrent, sans être limitatifs, l'encapsulation d'enzymes permettant de modifier le métabolisme de certains sujets présentant des anomali es acquises ou congénitales, ou pour l'encapsulation d'enzymes de détoxification.

Conformément à ce qui a été indiqué plus haut sur la possibilité, grâce au produit de l'invention de cibler vers le macrophage une substance active, il est particulièrement avantageux d'utiliser comme substance active à inclure dans les hématies un dérivé ou analogue du MDP puisque l'on sait que ces substances exercent leur actixité notamment par l'intermédiaire des macrophages (C. Leclerc et L. Chedid "Macrophage activation by synthetic muramyl peptides Jn Lymphokines, E.Pick Ed. Academic Press Inc. 7,1-21,1982). Parmi ces dérivés et analogues du MDP citons notamment ceux qui ont fait l'objet des demandes de brevet français sulvantes:

- 2 292 486
- 2 324 884
- 2 288 527
- 2 343 483
- 2 369 292
- 2 355 505
- 2 428 051

Parmi ces produits citons particulièrement les MDP dont la chaîne peptidique a été mono- ou di-estérifiée sur les groupements carboxyliques de l'acide D-glutamique par des chaînes alkyls de 1 à 10 carbones, de préférence de 1 à 6 carbones, en particulier l'ester butylique de la D-glutamine. Parmi les dérivés du MDP particulièrement avantageux citons également les mono- et di-esters comportant en position alpha une chaîne alkyl de 4 à 10 carbones, de préférence 4 carbones et en position gamma une chaîne alkyl inférieure de 1 à 3 carbones.

Bien entendu, il est possible, si cela est nécessaire et/ou utile, d'encapsuler plusieurs composés à activité biologique.

L'encapsulation de ces produits permet pour certains d'entre eux un ciblage sélectif sur certains organes ou bien l'induction d'un effet retard prolongé qui peut atteindre 15 à 25 jours ou plus selon les données actuelles recueillies chez l'animal.

Il est également important de noter que l'encapsulation peut induire un effet protecteur pour certains médicaments qui sont toxiques à l'égard de la plupart des tissus et des organes non visés par le médicament. Une activité de même type est observée dans l'induction d'un effet protecteur à l'égard du médicament ou de l'hormone transportée qui, dans certains cas, sont susceptibles d'induire une réponse immunitaire, ou bien lorsqu'elles sont injectées à des individus qui possèdent un anticorps dirigé contre ces substances dans la circulation sanguine, par exemple un anticorps anti-insuline. Cet effet protecteur peut aussi s'exercer à l'égard d'une dégradation métabolique du médicament ou d'hormone.

La stabilité intraérythrocytaire pourrait éventuellement nécessiter l'addition d'inhibiteurs de protéases comme dans le cas de l'insuline ou d'autres inhibiteurs enzymatiques.

Enfin, le procédé permet d'introduire des composés protecteurs de l'hémoglobine et de la membrane permettant, notamment, de protéger l'intégrité des érythrocytes.

Il peut s'agir de cryoprotecteurs ou de lyoprotecteurs; ces derniers intervenant en particulier pour la cryodessication que le procédé permet d'envisager.

Il faut enfin noter que les hématies peuvent être congelées lysées et être rescellées à la décongélation, ou en cas de lyophilisation, au cours des phases de réhydratation. Cette dernière situation permet de résoudre théoriquement le passage transmembranaire de l'eau lors de la réhydratation après lyophilisation.

La présente invention concerne également un dispositif destiné à l'encapsulation dans les érythrocytes humains ou animaux d'au moins une substance présentant une activité biologique, caractérisé en ce qu'il comporte en combinaison au moins:

- un élément de dialyse comportant au moins un compartiment primaire et un compartiment secondaire séparés par une paroi de dialyse,

- un dispositif d' alimentation en continu de la suspension d'érythrocytes dans le compartiment primaire dudit élément de dialyse,

- un moyen permettant d' amener une solution aqueuse dans le compartiment secondaire de l'élément de dialyse,

- un moyen assurant l'introduction de la substance à activité biologique dans le compartiment primaire de l'élément de dialyse,

- un moyen assurant le transfert de la solution effluente du compartiment primaire dans un ensemble de rescellement comportant au moins un moyen pour augmenter la pression osmotique et/ou oncotique de la solution effluente.

En ce qui concerne l'élément de dialyse, sa structure ne constitue pas en soi une caractéristique du dispositif selon la présente invention et il est possible d'utiliser tout type d'élément de dialyse comme cela a été indiqué précédemment dans le cadre du procédé.

Comme dispositif d'alimentation en continu, on peut envisager un dispositif quelconque, notamment un système de pompe, en particulier les pompes péristaltiques couramment utilisées, dans le domaine des dialyses.

Le dispositif selon la présente invention peut comporter différents modes. de réalisation, en particulier au niveau de l'ensemble de rescellement.

Le premier mode de réalisation de l'ensemble de rescellement peut être constitué par un élément de dialyse comportant au moins un compartiment primaire et un compartiment secondaire séparés par une paroi de dialyse, la solution effluente étant amenge dans le compartiment primaire par le moyen de transfert et le moyen pour augmenter la pression osmotique étant constitué par une solution hypertonique par rapport à la solution effluente, ladite solution hypertonique étant contenue dans le compartiment secondaire de l'élément de dialyse.

Mais il est également possible de prévoir d'autres modes de réalisation de cet ensemble de rescellement qui peut être constitué par une enceinte comportant un moyen d'amenge d' une solution hypertonique par rapport à la solution effluente.

Comme la lyse est effectuée de préférence à température assez basse, comprise entre 0 et 10°C, et de préférence aux environs de 4°C, et comme le rescellement est effectué de préférence à une température plus élevée, par exemple entre 20 et 40°C, le dispositif selon l'invention comportera, de préférence, un moyen de chauffage de la suspension à traiter, le moyen de chauffage pouvant être situé entre l'élément de dialyse et l'ensemble de rescellement ou bien directement à l'intérieur de l'ensemble de rescellement.

Dans un autre mode de réalisation, il est possible de prévoir après le premier élément de dialyse un récipient permettant de recueillir l'ensemble du volume de suspension à traiter, puis après avoir changé la tonicité de la solution aqueuse circulant dans le compartiment secondaire de l'élément de dialyse, de recycler le lysat d'érythrocytes ainsi obtenu dans le même élément de dialyse, dans ce cas cet élément de dialyse est utilisé dans un premier temps pour la lyse de la suspension d'érythrocytes, dans un deuxième temps comme ensemble de rescellement.

Le dispositif selon la présente invention peut également comporter des circuits d'asservissement qui, en réponse à des modifications de caractéristiques de la suspension traitée, agiront sur l'ensemble des paramètres de la réaction afin de ramener les caractéristiques de la suspension au niveau d'un seuil de consigne déterminé.

D'autres caractéristiques et avantages du procédé et dispositif selon la présente invention pourront être dégagés dans la description des figures ci-après sur lesquelles:

- la figure 1 représente un premier mode de réalisation du dispositif selon la présente invention,

- la figure 2 représente un second mode de réalisation du dispositif selon la présente invention,

- la figure 3 représente un troisième mode de réalisation du dispositif selon la présente invention,

- la figure 4 représente un quatrième mode de réalisation du dispositif selon la présente invention,

- la figure 5 représente la pression partielle d'oxygène en fonction du pourcentage de saturation des érythrocytes après encapsulation d'IHP

- la figure 6 est une photographie au microscope électronique d'une suspension érythrocytaire rescellée,

- la figure 7 représente le pourcentage d'hémolyse en fonction de la pression osmotique pour divers prélavages,

- la figure 8 représente les rendements bruts et nets en fonction d'un débit de sang pour divers prélavages selon la fig. 7,

- la figure 9 représente la quantité de Desféral ® encapsulée en fonction de la concentration initiale du produit,

7

- la figure 10 représente l'influence de la température de rescellement sur la fragilité osmotique des hématies rascellées,
- la figure 11 représente la variation de la $p_{50}$ pour diverses concentrations en IHP,
- la figure 12 représente les variations des diverses pressions d'oxygène sanguin,
- la figure 13 représente l'évolution du débit cardiaque mini-porc en fonction de la variation de la $p_{50}$ du sang transfusé,
- la figure 14 est une microphotographie de culots témoin et rescellé,
- la figure 15 représente une analyse comparative des histogrammes des volumes érythrocytaires.

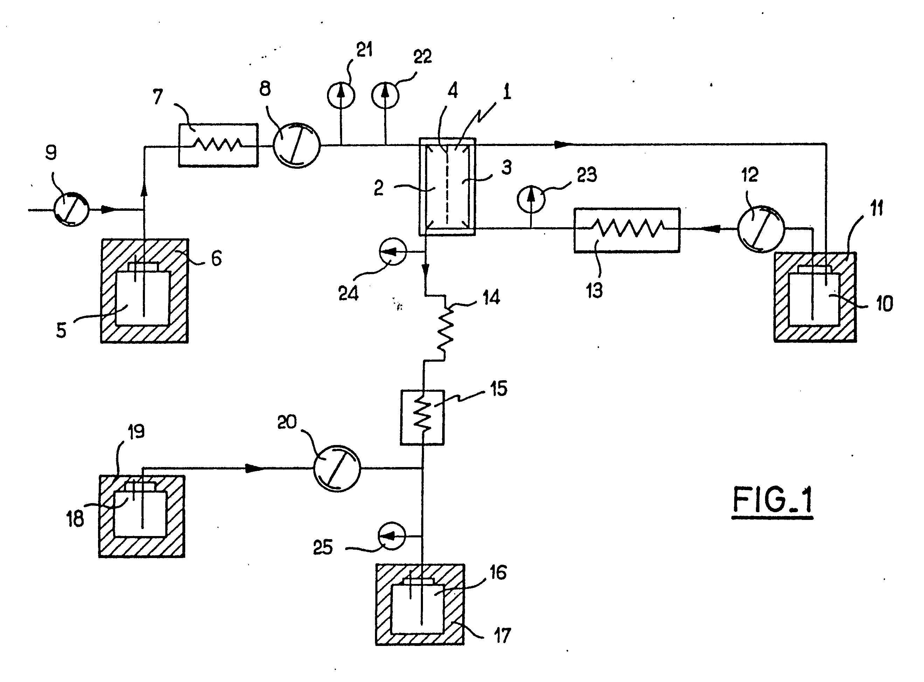

Le dispositif de la figure 1 est constitué d'un élément de dialyse 1 comportant un compartiment primaire 2 et un compartiment secondaire 3 séparés par une paroi de dialyse 4.

Le compartiment primaire 2 est alimenté par une suspension d'érythrocytes contenue dans le réservoir 5 placé dans l'incubateur 6.

Le sang avant d'être introduit dans le dialyseur 1 est amené à la température de lyse, en général 4 °C, par l'intermédiaire d'un échangeur de température 7.

La suspension d'érythrocytes est mise en circulation par l'intermédiaire de pompes péristaltiques telles que 8.

Dans le mode de réalisation décrit, le composé à activité biologique est introduit dans le circuit par l'intermédiaire de la pompe péristaltique 9 avant que la suspension d'érythrocytes ne pénètre dans l'échangeur de température 7.

Le compartiment secondaire 3 de l'élément de dialyse est alimenté en une solution hypotonique contenue dans le récipient 10 placé dans l'incubateur 11. Cette solution hypotonique est amenée dans le compartiment secondaire de l'élément de dialyse par l'intermédiaire d' une pompe péristaltique 12 et à travers un échangeur de température 13. Après son passage dans le compartiment secondaire de l'élément de dialyse, la solution hypotonique retourne au récipient 10; elle peut aussi être éliminée.

Afin de s'assurer que la lyse des érythrocytes est complète, il est possible, comme cela est représenté à la figure 1, de placer à la sortie de l'élément de dialyse 1 une ligne retard 14, c'est-à-dire le plus souvent une simple tuyauterie de grande longueur par exemple.

Dans le cas le plus fréquent où l'opération de rescellement doit être effectuée à une température supérieure à la température de lyse, on introduit après la ligne retard un échangeur de température tel que 15 qui permettra d'amener la suspension d'érythrocytes lysée à la température, par exemple, de 37°C.

Le lysat est placé en continu dans un récipient 16 contenu dans un incubateur 17, c'est dans ce récipient 16 que l'on ajoute en continu, ou bien en une seule fois, la solution de rescellement placée dans le récipient 18 contenu dans l'incubateur 19 par l'intermédiaire de la pompe péristaltique 20.

Lorsque le rescellement est complet, la suspension d'érythrocytes dans laquelle se trouve encapsulé le composé à activité biologique est prélevée directement à partir du récipient 16.

L'un des avantages du dispositif selon la présente invention est qu'il est possible de contrôler en permanence tous les paramètres de la réaction.

Bien entendu, il est possible de contrôler la température des différents fluides circulant à l'aide de dispositifs qui n'ont pas été figurés car ils sont bien connus de l'état de la technique.

Mais il est, en outre, très intéressant de pouvoir contrôler la pression des fluides et également leur pression osmotique afin de s'assurer que les échanges se feront dans les conditions optimales.

Il n' est bien entendu pas nécessaire de rentrer dans le détail de ces dispositifs de contrôle qui sont de type connu. On prévoiera, de préférence, au moins un manomètre tel que 21 pour contrôler la pression des fluides dans le circuit primaire et un élément de, mesure de la pression osmotique, par exemple par mesure de la conductivité à l'aide d'un dispositif tel que 22, ces éléments permettant d'agir pour modifier les autres paramètres de la réaction tels que le débit d'alimentation, afin de ramener les valeurs de la pression et de la pression osmotique à des valeurs de consigne.

Il en ira de même sur le circuit secondaire où un dispositif schématisé 23 permettra de mesurér la pression du fluide ainsi que la pression osmotique afin de maintenir les caractéristiques des fluides à certaines valeurs de consigne.

Il est représenté également d'autres dispositifs de contrôle tels que 24 et 25 assurant des fonctions identiques à celles définies précédemment.

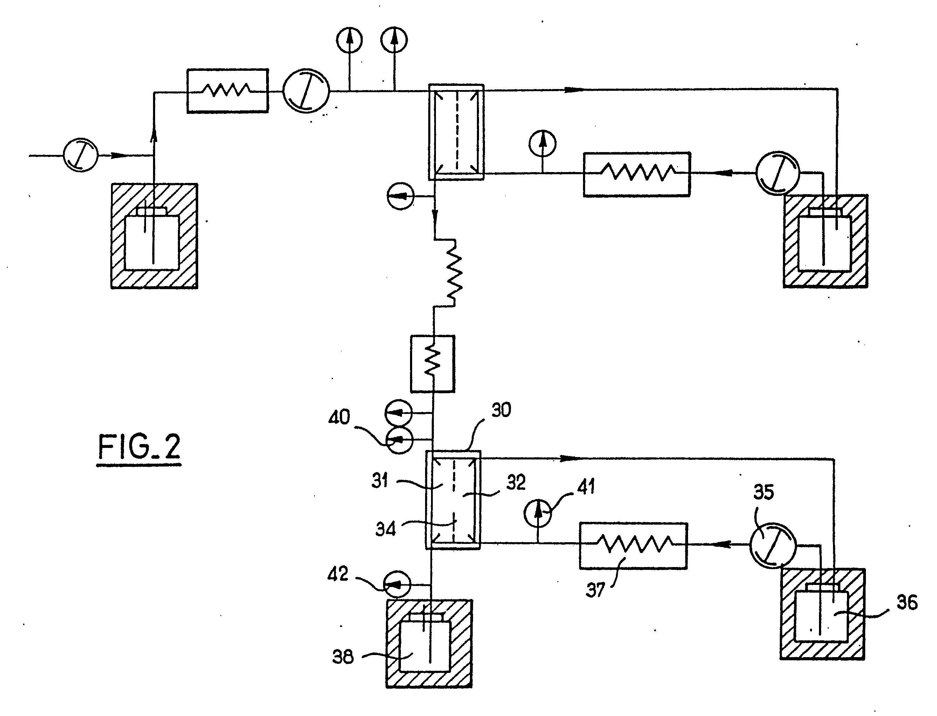

La figure 2 représente un dispositif qui est identique à celui de la figure 1 jusqu'au niveau du second échangeur thermique référencé 15 sur la figure 1, c'est pourquoi cette partie du dispositif ne sera pas redécrite puisqu'elle est absolument identique à celle de la figure 1.

Par contre, pour ce qui concerne l'étape de rescellement, celle-ci est effectuée par l'intermédiaire d'un second élément de dialyse 30 comportant un compartiment primaire 31 et un compartiment secondaire 32 séparés par une membrane de dialyse 34.

Une solution de rescellement hypertonique circule dans le compartiment secondaire 32 par l'intermédiaire d'une pompe péristaltique 35 qui prélève cette solution hypertonique dans le récipient 36 et la conduit par l'intermédiaire de 1' échangeur de température 37 dans le compartiment secondaire de l'élément de dialyse. Cette solution hypertonique peut être recyclée dans le récipient 36.

La suspension de lyse provenant du premier élément de dialyse, et après son passage dans la ligne retard et dans l'échangeur de température, est amenée dans le compartiment primaire de l'élément de dialyse 30 dans lequel les érythrocytes sont rescellés, la suspension après rescellement étant recueillie dans un récipient 38 contenu dans un incubateur.

Là encore, tout comme cela a été mentionné précédemment, on prévoit différents dispositifs de contrôle tels que 40, 41 et 42 permettant de contrôler en continu les paramètres de mise en oeuvre du procédé.

De façon générale, le débit du circuit primaire contenant les érythrocytes est compris entre 20 et 80 ml/min. alors que les débits des solutions hypertoniques ou hypotoniques du circuit secondaire sont de l'ordre de 100 à 1 000 ml/min.

Dans les essais qui seront rapportés ci-après, les surfaces de dialyseurs mis en oeuvre sont en général de l'ordre de 0,41 m$^2$

Les pressions dans les circuits sont voisines de 133. 10$^2$ Pa (100 mm de Hg) selon les débits utilisés.

Les forces ioniques des solutions de lyse sont d'environ 40 mosmoles et les forces ioniques des solutions de rescellement sont d'environ 300 mosmoles.

Le schéma de la figure 2 qui est symétrique pour les deux opérations de lyse et de rescellement montre qu'il est possible d'envisager l'utilisation d'un dispositif ne comportant qu'un seul élément de dialyse et de le faire fonctionner dans un premier temps pour lyser la suspension d'érythrocytes puis, après avoir recueilli l'ensemble de la solution lysée, de faire fonctionner cette bobine de dialyse pour le rescellement en changeant uniquement la tonicité de la solution alimentant le compartiment secondaire.

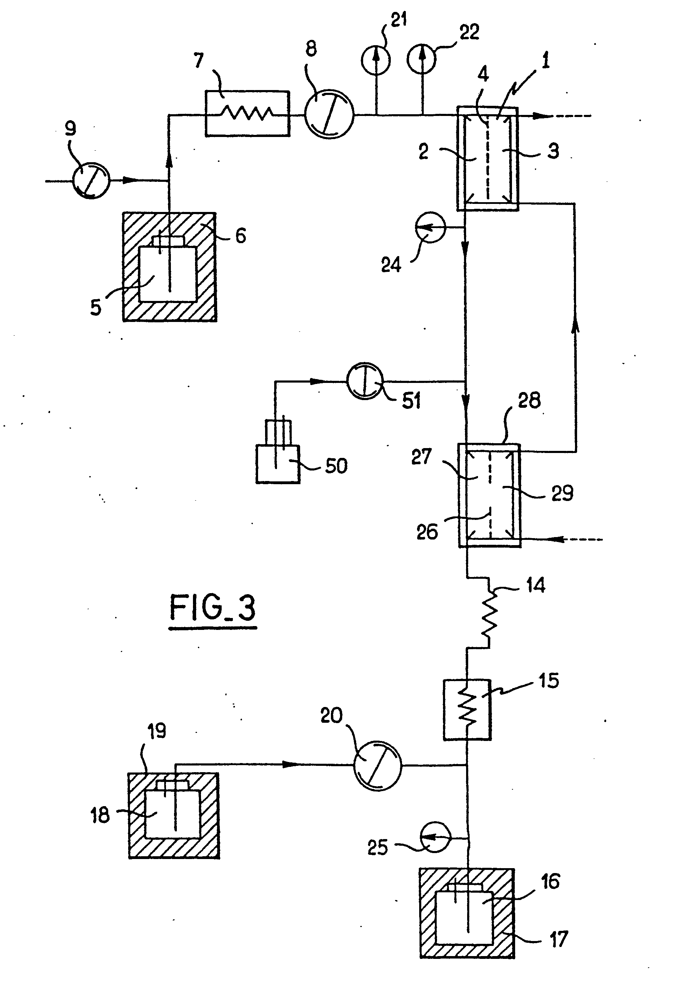

Le schéma de la figure 3 permet d'amener la pression osmotique de la suspension d'érythrocytes à 200 mos afin de limiter la perte de la substance à encapsuler comme cela a été indiqué précédemment.

La taille de l'élément de dialyse 1 est choisie pour que, compte tenu des débits et des volumes, l'abaissement de la force ionique dans la suspension érythrocytaire amène celleci à environ 200 mos. La substance à encapsuler contenue dans le récipient 50 est introduite à l'aide la pompe 51 dans le circuit de sortie du dialyseur 1. Ceci peut être simplement réalisé à l'aide d'un pousse-seringue à débit continu classique.

La suspension érythrocytaire issue du dialyseur 1 reçoit alors en continu la substance à encapsuler et pénètre dans le circuit primaire 27 d'un élément de dialyse 28 dont la membrane 26 possède une surface choisie, de telle sorte que soit alors effective la lyse des hématies passant dans le circuit primaire selon le procédé initial.

Les circuits secondaires 3 et 28 peuvent être alimentés en série par la même solution hypotonique telle qu'elle est indiquée sur la figure 1.

Le rapport des surfaces des membranes 1 et 26 est choisi pour optimiser les échanges moléculaires dans les deux phases de dialyse.

Les éléments de dialyse 1 et 28 peuvent être soit séparés pour utiliser des éléments commerciaux existants, soit intégrés dans un dialyseur ü deux compartiments spécialement adapté au procédé, ce qui est facilement réalisable selon la méthodologie actuelle.

La sortie du dialyseur 28 reprend les éléments du schéma de principe de la figure 1.

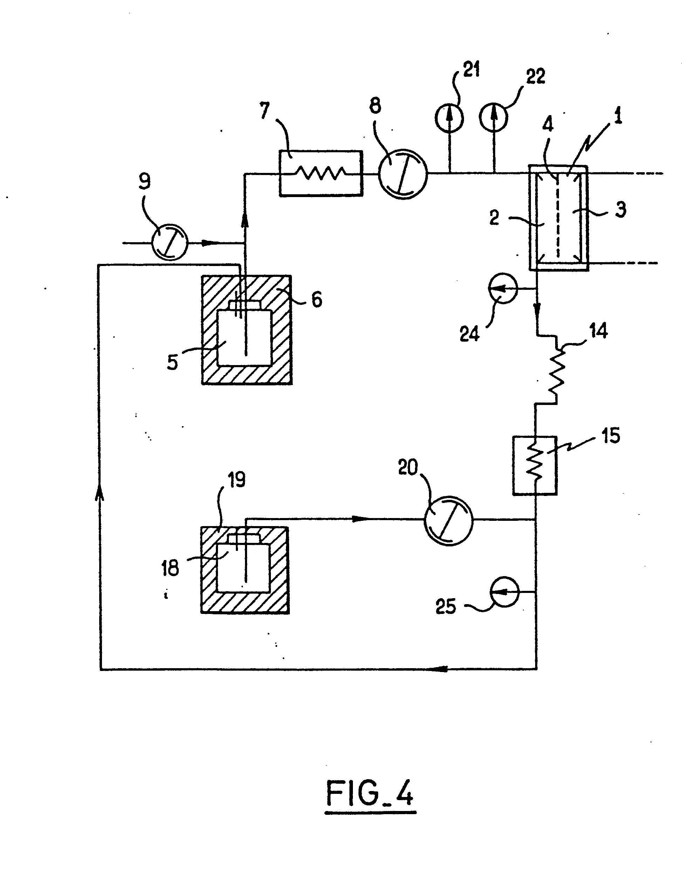

Le schéma de la figure 4 représente un mode de réalisation particulier du procédé selon l'invention.

Les conditions décrites jusqu'à présent sont particulièrement adaptées à l'encapsulation d'une substance dans un volume globulaire d'environ 200 à 400 ml ou à l'utilisation d'une transformation en flux continu. En modifiant surfaces des dialyseurs et débits, des volumes variables peuvent être traités.

Il est cependant utile de détailler un circuit de recyclage continu pour les conditions extrêmes:

a) La transformation d'un volume d'une suspension érythrocytaire de plusieurs litres, si le rescellement n'est pas effectué en continu, introduit une différence importante dans la cinétique du phénomène pour les hématies qui sont passées les premières dans les phases de dialyse par rapport à celles qui passeront les dernières.

b) De la même façon, la manipulation à l'aide d'un minidialyseur de faibles quantités de sang (quelques ml) pour l'encapsulation de substances très actives qui ne necessitent pas des volumes importants de sang pour les doses à administrer, introduit un paramètre supplémentaire. En effet, l'efficacité d'un dialyseur dépend de sa surface, donc permet un équilibre de dialyse déterminé, mais cet équilibre n'est atteint que selon une cinétique d'autant plus lente que la surface est réduite. Une simple adaptation débit/surface est donc insuffisante pour réaliser une encapsulation dans des conditions convenables.

Dans ce mode de réalisation, la surface du dialyseur 1 est réduite et le débit du sang dans le circuit primaire augmenté de sorte qu'à la sortie du dialyseur 1 la pression osmotique n'est réduite que pour une fraction de ce qui serait nécessaire pour provoquer la lyse des globules rouges. La suspension erythrocytaire est recyclée en continu dans le réservoir initial 5. L'échangeur de température 15 peut être programmé uniquement en fin de transformation, de même pour la pompe 20.

Ce dispositif permet d'effectuer la mise en oeuvre du procédé par modifications successives et progressives des forces ioniques des milieux, ce qui permet de négliger la cinétique propre du dialyseur 1, en faisant intervenir une cinétique complémentaire liée au volume de sang à traiter et au débit dans le circuit.

Cette modification permet d'adapter facilement le procédé à une gamme très large de volumes de sang traités, compte tenu des caractéristiques techniques des dialyseurs actuellement utilisables et donc, en principe, de rechercher les conditions optimales d'encapsulation pour des conditions très variées. On peut, ainsi, transformer aussi bien de grands volumes que quelques ml de sang destinés à une expérimentation animale ou pour l'encapsulation d'une substance rare et onéreuse.

Les dispositifs schématisés aux figures 1 et 2 ont été utilisés afin de mettre en oeuvre le procédé revendiqué.

## EXAMPLE 1

Incorporation de desferrioxamine (Desféral®)

Dans cet exemple, on utilise le dispositif schématisé à la figure 1.

Le dialyseur 1 présente une surface de 0,41 m$^2$ et est alimenté par une suspension d' érythrocytes avec un débit de 60 ml/min dans le compartiment primaire alors que dans le compartiment secondaire on fait passer, avec un débit de 500 ml/min, une solution de lyse comportant NaH$_2$PO$_4$-Na$_2$HPO$_4$, pH 7,4, 10 mM, CaCl$_2$ 0,5 mM et 2mM de glucose.

La suspension d'érythrocytes a été obtenue de la façon suivante: A 200 ml de culot globulaire lavés, à 70 % d'hématocrite, on ajoute 5 ml de solution contenant 250 mg de Desféral®. La lyse est effectuée à 4°C en maintenant la suspension de lyse dans un récipient 16 à cette température pendant 10 min. On introduit ensuite 20 ml de NaCl 1,5 M, contenant 10 % de PEG 4000. La suspension est maintenue en contact avec cette solution de rescellement pendant 30 min à 37°C. Ensuite, on prélève l'ensemble et on effectue 3 lavages par NaCl à 9 g/l.

Il est utile d'effectuer un lavage intermédiaire par une solution à 200 mos ou 250 mos environ pour éliminer certaines populations érythrocytaires fragilisées. De même, la resuspension dans le plasma initial ou dans des solutions de régénération peut se révéler nécessaire pour une bonne conservation des hématies.

Après dosage, on constate que l'on a incorporé dans le culot globulaire 148 mg de Desféral® (soit un rendement brut de 59 %), mais il convient de remarquer que 12 mg de Desféral® ont été éliminés dans la solution de dialyse, ce qui conduit en fait à un rendement net d'incorporation du Desféral® dans les érythrocytes de 63,5 %.

Si l'on modifie le débit d'alimentation en suspension d'érythrocytes pour l'amener à 80 ml/min, le rendement brut d'incorporation est de 47,3 % et le rendement net est de 57,9 %.

Pour un débit de 40 ml/min le rendement brut est de 49,4 % et le rendement net de 59,1 %.

Pour un débit de 20 ml/min le rendement brut est de 46,1 % et le rendement net de 64,2 %.

Le rendement brut est calculé par rapport au produit total mis en réaction et le rendement net est calculé par rapport au produit restant après la phase de lyse.

Ces résultats démontrent qu' il existe un optimum de débit de suspension érythrocytaire pour une surface de dialyseur donnée, lorsque la substance qui doit être incorporée est une substance susceptible de diffuser à travers la membrane de dialyseur.

## EXEMPLE 2

### Incorporation de desferrioxamine (Desféral®)

Dans cet exemple, on met en oeuvre le dispositif décrit à la figure 2, c'est-à-dire que l'on effectue le rescellement à l'aide d' une deuxième dialyse à 22°C à l'aide d'un élément de dialyse tel que 30.

Dans ce cas, on utilise le tampon de rescellement suivant: NaH$_2$PO$_4$-Na$_2$HPO$_4$ 10 mM, pH 7, 4, CaCl$_2$ 0'5 mM, glucose 2 mM, NaCl 144 mM.

Les autres paramètres restant identiques, le débit de suspension érythrocytaire est de 50 ml/min.

Dans ce cas, on observe un rendement net d'incorporation de 46,7 % alors que le rendement brut est de 32,5 %.

Ces résultats s'expliquent aisément puisque le Desféral® est une substance diffusible et que l'on tend à perdre encore une nouvelle quantité de Desféral ® lors de la seconde dialyse.

## EXEMPLE 3

### Incorporation d'insuline

Dans cet exemple on encapsule de l'insuline dans les érythrocytes en mettant en oeuvre le dispositif schématisé à la figure 1. Dans ce qui suit il ne sera mentionné que les paramètres qui diffèrent de ceux indiqués à l'exemple 1.

Afin de permettre le dosage de l'insuline, le produit utilisé est marqué avec I$^{125}$ par la méthode de la chloramine T.

On ajoute 10 ml d'insuline (4 U/ml-432.800 cpm/ml) à 200 ml de culot globulaire lavés trois fois avec NaCl à 9 g/l.

Le débit de suspension érythrocytaire utilisé est de 60 ml/min. Après la lyse, l'incubation se poursuit 10 min à 4°C.

Le rescellement est effectué à 37°C à l'aide de 20 ml de NaCl 1,5 M contenant 10 % de PEG 4 000.

L'incubation est poursuivie 30 min à 37°C et les globules rouges sont ensuite lavés trois fois par NaCl à 9 g/l.

Dans ces conditions, le rendement d'incorporation de la radioactivité est de 48% (± 4 %). Bien entendu, cette incorporation ne saurait préjuger de l'activité biologique de l'insuline encapsulée dans les globules rouges. Il est bien connu que cette hormone est modifiée en, présence d'hémolysat de globule rouge.

Toutefois, ce résultat confirme les possibilités d'incorporer dans les érythrocytes une protéine de 7 000 daltons.

Afin de conserver l'intégrité fonctionnelle de l'insuline, il peut être nécessaire de prévoir d'incorporer des substances protectrices de l'insuline au milieu érythrocytaire, ceci peut bien entendu être réalisé très aisément à l'aide du dispositif selon la présente invention.

## EXEMPLE 4

### Incorporation d'albumine

On opère comme cela est décrit dans l'exemple 3 en utilisant de l'albumine marquée à $I^{125}$. Dans ces conditions, on observe un rendement qui est de l'ordre de 35 %, ce résultat montre l'influence de la masse moléculaire sur le rendement d'incorporation d'une protéine. > > >

Les exemples suivants sont destinés à illustrer l'encapsulation d'effecteurs allostériques de l'hémoglobine, notamment l'inositol hexaphosphate (IHP) grâce au procédé de la présente invention.

## EXEMPLE 5

### Encapsulation d'IHP

Pour réaliser une transformation plus ou moins importante des globules rouges à l'aide de l'inositol hexaphosphate, on utilise par exemple deux types de solution permettant de faire varier le rapport IHP/Na.

### Solution A

15 g d'IHP 12 Na (12% $H_2O$) dans 55 ml d'eau distillée sont passés sur une colonne de DOWEX 50 W-400 mesh équilibrée en ions $H^+$. On obtient 63 ml d'IHP acide ($14,2 . 10^{-3}$ mole).

Cet IHP acide est utilisé pour neutraliser une autre solution d'IHP 12 Na jusqu'à pH 7,4. $38,1 . 10^{-3}$ mole d'IHP 12 Na dans 153 ml sont neutralisés par 97 ml d'IHP acide contenant $17 . 10^{-3}$ mole d'IHP.

Au total on obtient une solution contenant $55 . 10^{-3}$ mole IHP et $457 . 10^{-3}$ mole sodium dans 250 ml. Cette solution est diluée pour obtenir un milieu à 250 mosmoles/l.

### Solution B

11 g d'IHP 12 Na (12% $H_2O$) sont neutralisés par 40 ml d' HCl N dans 750 ml d'$H_2O$ à pH 7,4. On obtient un milieu à 230 mosmoles/l.

Dans cet exemple, on utilise le dispositif schématisé à la figure 1.

Le dialyseur 1 présente une surface de 0,41 $m^2$ et est alimenté par une suspension d'érythrocytes avec un débit qui varie selon les expériences de 20 à 60 ml/min.

Le circuit secondaire est alimenté à 500 ml/min par un tampon de lyse ayant la composition suivante:
- $CO_3HNa$ 10 mM
- $PO_4H_2K/PO_4HK_2$ 10 mM,
- glucose 2 mM,
pH 7,4.

La suspension d'érythrocytes utilisée a été obtenue de la façon suivante:

A 200 ml d'un culot globulaire d'érythrocytes lavés 3 fois par une solution de NaCl à 9 g/l (70 % d'hématocrite), on ajoute un volume équivalent de l'une des solutions A ou B.

Après centrifugation, le surnageant légèrement hémolysé est éliminé.

La lyse est effectuée à 4°C. La suspension de lyse est maintenue dans un récipient 16 pendant 10 minutes à 37°C en présence d'IHP.

La suspension de lyse est rescellée par addition de 10 % en volume d'une solution contenant NaCl 1,5 H, PEG 4000 à 10 %.

Après incubation de 30 minutes à 37°C, les culots globulaires sont lavés 3 fois par une solution de NaCl à 9 g/l et remis en suspension dans le plasma d'origine.

Il est utile d'effectuer un lavage intermédiaire par une solution à 220 mos environ afin d'éliminer certaines populations érythrocytaires fragilisées.

De même, la resuspension dans le plasma initial ou dans des solutions de régénération peut se révéler nécessaire pour une bonne conservation des hématies.

Les résultats de ces expériences sont rassemblés dans le tableau ci-après:

| Traitement | Témoin | Lyse NaCl rescellés sans IHP | Solution A | | | Solution B | | |
|---|---|---|---|---|---|---|---|---|
| Débit (ml/min) | | 60 | 60 | 40 | 20 | 60 | 40 | 20 |
| $P_{50}$ (mm Hg) | (20,6) | (19,6) | (51,5) | (66,5) | (77,0) | (36,8) | (40,7) | (46,5) |
| $10^2$ PA | 27,4 | 26,1 | 68,6 | 88,6 | 102,6 | 49,0 | 54,2 | 61,9 |
| $n$ de Hill 30—70 (mm Hg)$^{-1}$ | (2,40) | (2,50) | (1,64) | (1,97) | (1,25) | (1,49) | (1,53) | (1,47) |
| $10^{-2}$ Pa$^{-1}$ | 1,80 | 1,87 | 1,23 | 1,47 | 0,93 | 1,11 | 1,14 | 1,10 |

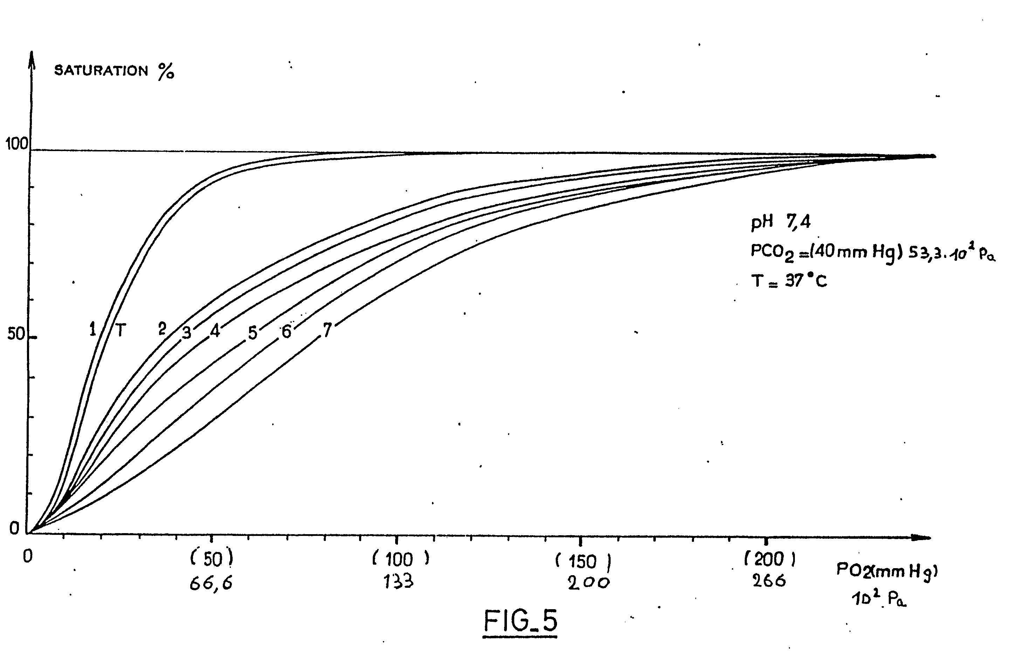

Les courbes de liaison de l'oxygène représentées à la figure 5 sont mesurées à 25°C par la méthode de diffusion rapide à l'aide de l'appareil de Duvelleroy (J. Appl. Physsiol. 28, 227-233 (1970) et Tesseirre et al. (Bull. Physiopath. Resp. 11, 837-851 (1975).

La courbe de la figure 5 représente pour divers débits de suspension érythrocytaires et suivant la nature de la solution utilisée, A ou B, la pression partielle d'oxygène en Pa (mm de Hg) en fonction du pourcentage de saturation des érythrocytes.

La pression de demi-saturation notée dans le tableau $P_{50}$ est une indication de l'affinité de l'oxygène pour l'hémoglobine. La pente de la courbe au voisinage de la demisaturation, c'est-à-dire entre 40 et 60 %, représente le degré de coopération des quatre sites de fixation de l'oxygène dans l'hémoglobine, cette pente permet de calculer le coefficient de HILL.

On détecte une amélioration de la libération de l'oxygène, c'est-à-dire une diminution de l'affinité de l'hémoglobine pour l'oxygène, soit par une diminution du coefficient de HILL, c'est-à-dire une diminution de la pente de la courbe de la figure 5 au voisinage de 50 % de la saturation, ou bien par un déplacement global de la courbe vers la droite qui correspond à une augmentation de la pression partielle d' oxygène au voisinage de 50 % de la saturation.

Pour les érythrocytes témoins de la courbe T dont la $P_{50}$ est de $25,7.10^2$ Pa (19,3 mm de Hg), on observe que les mêmes érythrocytes traités par le procédé de l'exemple en présence initiale de sérum physiologique ont une $P_{50}$ de $24,6.10^2$ Pa (18,5 mm de Hg) alors qu'apres incorporation d' IHP par le procédé selon la présente invention les $P_{50}$ observées varient entre 49 et $102.10^2$ Pa (38,8 et 77 mm de Hg) suivant les débits utilisés ainsi que la nature de la solution d'IHP utilisée.

## EXEMPLE 6

En utilisant la solution A diluée pour obtenir 210 mosmoles/l on effectue une lyse à 4°C avec un débit de 20 ml/min puis un rescellement de 30 min en faisant varier la température. Le tableau ci-après montre l'influence de la température de rescellement sur la valeur de la $P_{50}$ obtenue mais aussi sur le rendement en globules rouges reconstitués exprimé en faisant le rapport des hématocrites des suspensions de globules rouges reconstitués à l'hématocrite de la suspension initiale pour un volume total équivalent.

| Température de rescellement | $P_{50}$ | Rendement (hématocrite) |
|---|---|---|
| 25°C | $55.10^2$Pa (42 mm Hg) | 27 % |
| 30°C | $63.10^2$Pa (48 mm Hg) | 36 % |
| 35°C | $67.10^2$Pa (50,5 mm Hg) | 45 % |
| 40°C | $72.10^2$Pa (54,5 mm Hg) | 50 % |

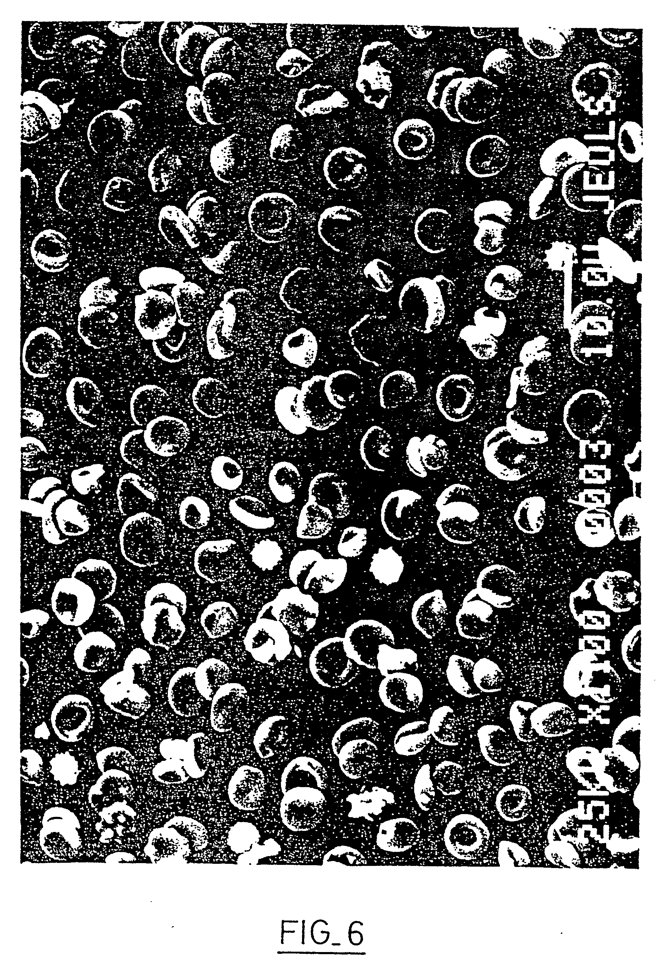

· Après rescellement et maintien en milieu plasmatique, la plus grande partie de globules rouges retrouve une forme discocytaire bien que faiblement microcytaire. Les formes plus ou moins vésiculaires ne sont pas significativement plus nombreuses que dans la population native. Ceci est confirmé par la figure 6 correspondant à une photographie au microscope électronique à balayage d' une suspension érythrocytaire rescellée dont la $P_{50}$ est de $90.10^2$. Pa (68 mm de Hg).

Les conditions expérimentales décrites démontrent les grandes possibilités d'utilisation de la dialyse en flux continu pour réaliser dans des conditions variables l'incorporation d'IHP ou d'autres effecteurs allostériques de l'hémoglobine afin de réaliser une modification des propriétés oxyphoriques du sang.

Le contrôle des paramètres de la réaction de lyse-rescellement, volumes, débits, températures, compositions des milieux, est aisément réalisable à l'aide du dispositif expérimental utilisé.

Ce dispositif permet la transformation rapide de quantités importantes de globules rouges. Il pourrait éventuellement être incorporé dans un circuit de circulation extracorporelle. Les conditions d'utilisation permettent une application thérapeutique chez l'homme.

La méthode d'incorporation d'effecteurs allostériques de l'hémoglobine dans les globules rouges décrite dans ce brevet présente une série d' avantages évidents par rapport à la méthode liposomale décrite dans le brevet des Etats-Unis d'Amérique n° 4 192 869:

1) Elle n'utilise pas de phospholipides et du cholestérol exogènes et en cela simplifie considérablement les problèmes de toxicité ainsi que d' activités immunologiques acquises par les globules rouges.

2) Elle permet une quantification très précise de la concentration d'effecteurs allostériques de l'hémoglobine incorporée et par conséquent permet l'obtention de la $P_{50}$ souhaitée.

3) Le processus d'incorporation est plus rapide et largement automatisable.

4) La reproductibilité de cette méthode est supérieure à celle de la méthode liposomale.

Les exemples suivants réalisés avec le Desféral selon le processus de l'exemple 1 mettent en évidence l'influence de divers paramètres du procédé.

## EXEMPLE 7

### Influence d'un prélavage sur la courbe lyse

Comme cela a été décrit précédemment, on peut envisager de faire gonfler les hématies, avant d'effectuer la lyse, en effectuant un prélavage à l'aide d'une solution contenant une substance diffusible à travers la membrane du globule rouge comme par exemple le glucose.

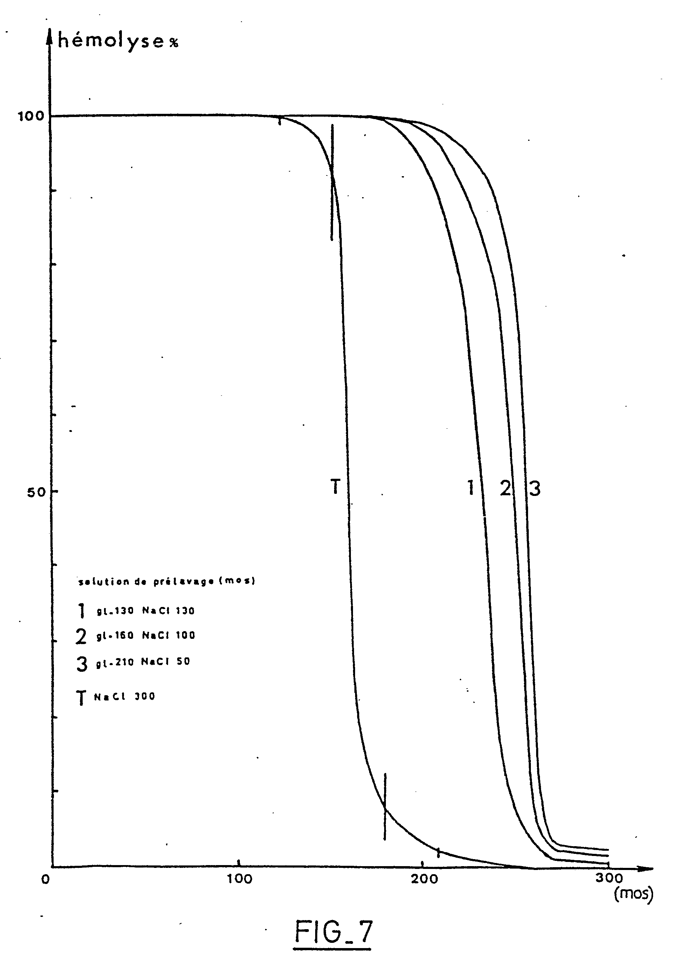

La figure 7 représente les courbes de lyse obtenues après lavages dans des solutions contenant des proportions variables de glucose et de chlorure de sodium.

### Solution de prélavage (mos)

1 – 130 glucose      130 NaCl

2 – 150 glucose      100 NaCl

3 – 210 glucose      50 NaCl

T –      300 NaCl.

Les courbes de la figure 7 démontrent clairement l'intérêt de faire gonfler les hématies afin d'améliorer la lyse, mais il faut tenir compte du fait que parallèlement le volume des hématies augmente. Pour un hématocrite de départ de 70 %, le volume extracellulaire après rescellement sera d'autant plus important que le volume globulaire moyen aura été artificiellement augmenté. Ces deux effets sont donc antagonistes sur le plan du rendement d'encapsulation final.

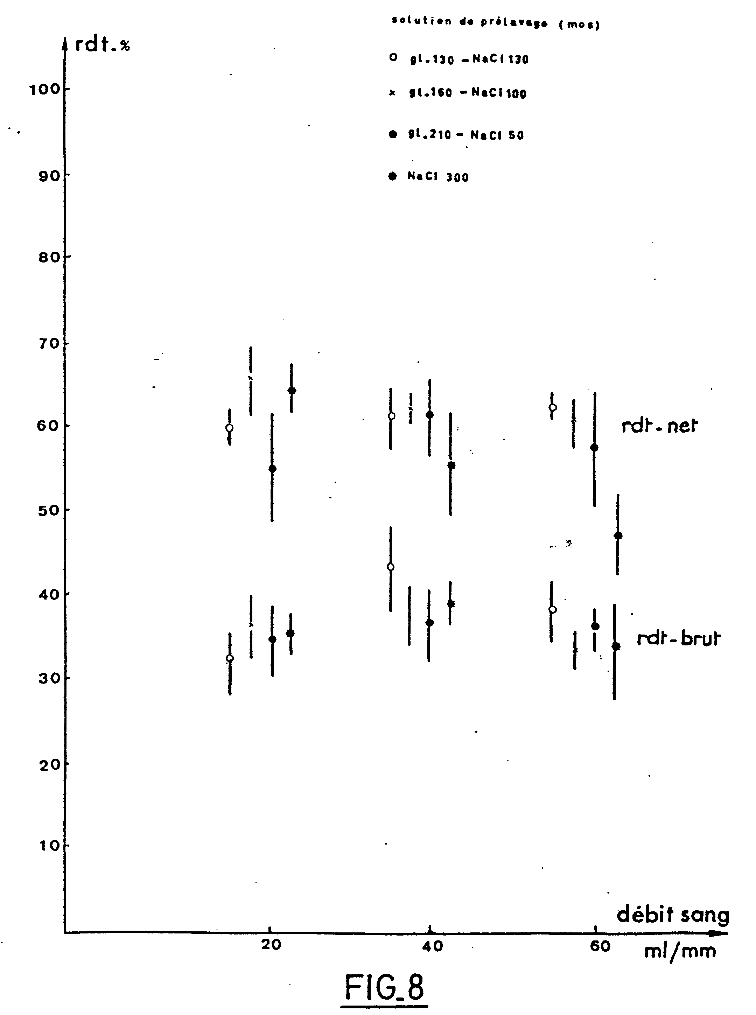

Il existe donc un optimum entre les conditions de prélavage et les conditions de lyse comme cela est illustré sur la figure 8.

La figure 8 représente, en effet, les rendements nets et les rendements bruts d'encapsulation pour divers débits de sang et pour les diverses solutions de prélavage et l'on constate que pour un débit de sang donné il existe, en général, au moins un prélavage qui permet d'augmenter les rendements.

Les résultats initiaux donnés pour l'encapsulation du Desféral dans les exemples 1 et 2 ont été obtenus à l'aide d'une méthode de dosage chimique colorimétrique. Ces résultats sont majorés par rapport à ceux obtenus à l'aide de la méthode de dosage radioactif utilisant le complexe $^{59}$Fe-Desferrioxamine utilisée pour l'obtention des résultats de la figure 8. Cette méthode est utilisée dans les exemples suivants:

Cet exemple montre qu'il est possible en ajustant les conditions expérimentales d'obtenir des résultats compris entre 40 et 50 % pour les rendements bruts.

## EXEMPLE 8

### Influence de la quantité de Desféral ajoutée

Dans cet exemple, on mesure la quantité de Desféral encapsulée pour diverses concentrations de Desféral dans le culot globulaire.

Les conditions expérimentales sont déterminées pour donner un rendement brut d'environ 40 %.

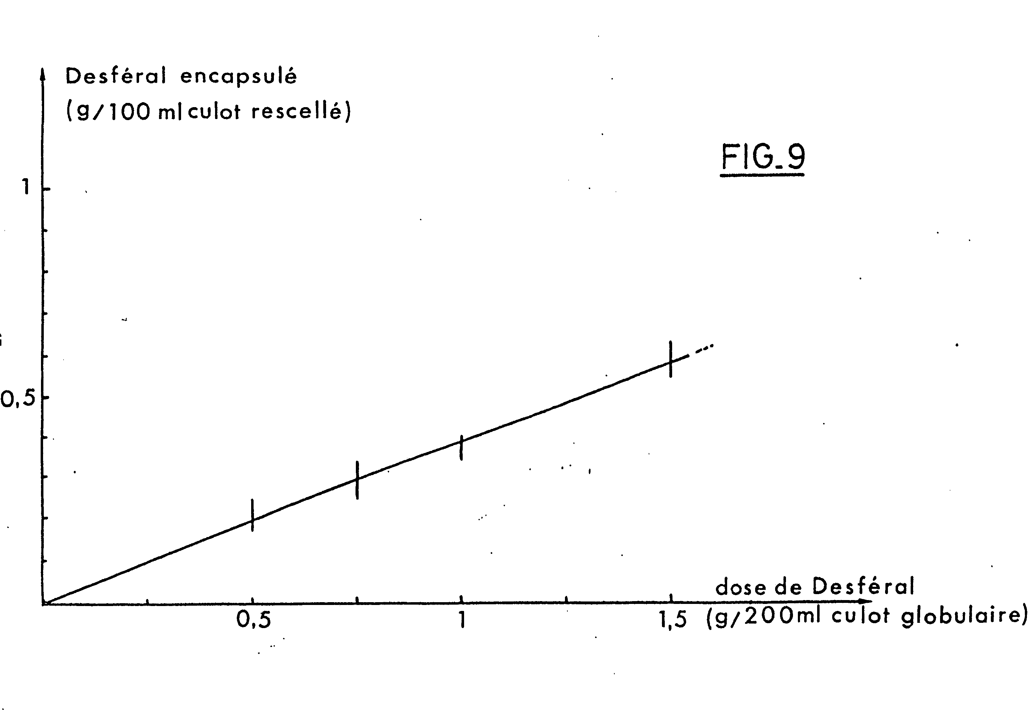

La figure 9 rassemble les résultats obtenus et montre l'existence d'une relation linéaire pour une large zone de concentration de Desféral.

De cette figure il ressort que la dose maximale de Desféral encapsulable n'est pas atteinte. En utilisant les conditions optimales de la figure, 8 ou celles correspondant à l'utilisation de deux dialyseurs mentionnées ci-après (rendement d'environ 50 %), on voit qu'il est possible d'encapsuler 800 mg à 1 g, au moins, de Desféral pour 100 ml de culot rescellé.

## EXEMPLE 9

### Influence de l'hématocrite

Le tableau suivant montre l'influence de l'hématocrite du culot globulaire lavé sur le rendement d'encapsulation.

| Hématocrite % | Rendement |
|---|---|
| 50 | 35,3 |
| 60 | 35,7 |
| 70 | 40,4 |
| 80 | 42 |

## EXEMPLE 10

### Influence de la température de rescellement

Le tableau de l'exemple 6 indique l'influence de la température sur le rendement de rescellement et d'encapsulation pour l'IHP.

Des essais complémentaires effectués avec le Desféral ont montré qu'entre 37 et 42°C l'influence de la température sur le rendement final est relativement modérée mais que, par contre, les courbes de lyse des hématies rescellées sont considérablement modifiées.

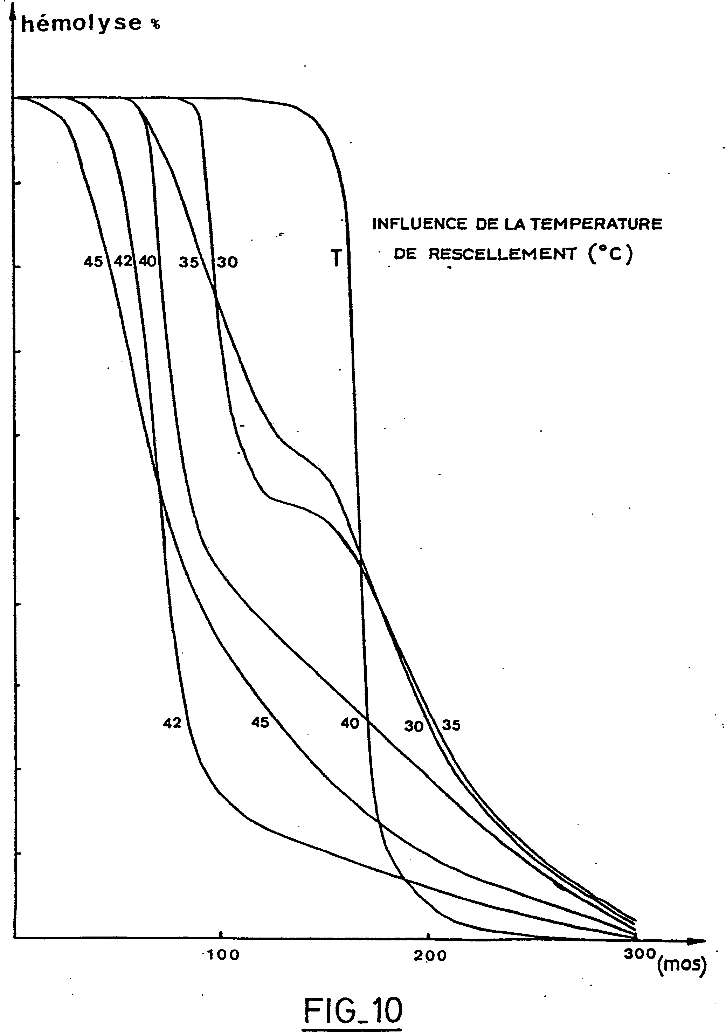

La figure 10 représente l'influence de la température de rescellement sur la lyse bactérienne en fonction de la tonicité du milieu.

Les formes les plus stables sont obtenues vers 40 à 42°C (déplacement des courbes vers la gauche). A partir de 45°C, une fragilisation des globules rouges apparaît (déplacement des courbes vers la droite).

## EXEMPLE 11

La composition de la solution de rescellement utilisée selon l'exemple 1 joue un rôle important, comme indiqué dans le tableau ci-dessous pour deux hématies diférentes:

| Composition de la solution | Rendement brut % | Rendement net % |
|---|---|---|
| NaCl 1 M | 42,7—40 | 70,7—63,1 |
| NaCl 1,2 M | 38,3—39,4 | 69,1—62,9 |
| NaCl 1,5 M | 38,8—37,3 | 66,6—57,3 |
| NaCl 1 M + PEG 4000 10 % | 42,1—41,6 | 66,9—63,4 |
| NaCl 1,2 M+PEG 4000 10 % | 39,7—37,8 | 69,9—62,6 |
| NaCl 1,5 M+PEG 4000 10 % | 38,3—37,3 | 68,3—61,2 |

Les dosages sont effectués selon la méthodé isotopique

Comme cela a été décrit précédemment, afin de respecter la durée de vie maximale des hématies in vivo, il est bien connu que certains constituants sont essentiels. Ce sera le cas de l'ATP, de l'équilibre Na/K, des ions $Ca^{2+}$ et $Mg^{2+}$ du glucose ou d'autres composés comme l'Inosine ou le 2-3 DPG par exemple.

Il peut, en conséquence, être nécessaire de modifier la composition de la solution de rescellement ou des solutions de lavages pour respecter au maximum l'intégrité biologique des hématies rescellées.

## EXEMPLE 12

Cet exemple a été mis en oeuvre en utilisant un dispositif conforme à celui de la figure 3, c'est-à-dire dans lequel la substance active est introduite entre deux phases de la lyse des globules rouges.

Les tableaux ci-dessous résument les résultats moyens correspondant à l'utilisation de ce dispositif pour l'encapsulation de Desféral.

Les paramètres sont:
- $S_1$ = surface du dialyseur 1
- $S_2$ = surface du dialyseur 28
- Débit du sang = 20, 40, 60 ou 80 ml/min pour différentes solutions de prélavage des hématies:
  a) solution de NaCl 0,15 M (300 mos)
  b) solution de NaCl 130 mos + glucose 130 mos.

### a) — Prélavage par NaCl 300 mos:

— $S_1$ = 0,28 m²        — $S_2$ = 0,28 m²

| débit ml/min | rdt net % | rdt brut % |
|---|---|---|
| 20 | 51,9 | 38,4 |
| 40 | 38,8 | 31,6 |
| 60 | 27,7 | 24,7 |
| 80 | 19,3 | 17,7 |

— $S_1$ = 0,28 m²        — $S_2$ = 0,41 m²

| débit ml/min | rdt net % | rdt brut % |
|---|---|---|
| 20 | 51,6 | 32,3 |
| 40 | 39,6 | 28,7 |
| 60 | 36,6 | 28,3 |
| 80 | 38,1 | 27,5 |

— $S_1$ = 0,41 m²        — $S_2$ = 0,28 m²

| débit ml/min | rdt net % | rdt brut % |
|---|---|---|
| 20 | 50,75 | 46,2 |
| 40 | 50,57 | 39,5 |
| 60 | 42,4 | 39,2 |
| 80 | 34,4 | 32,6 |

### b) — Prélavages NaCl 130 mos + glucose 130 mos:

— $S_1$ = 0,28 m²        $S_2$ = 0,28 m²

| débit ml/min | rdt net % | rdt brut % |
|---|---|---|
| 20 | 51,8 | 46,8 |
| 40 | 52 | 45,75 |
| 60 | 48,5 | 42,1 |
| 80 | 51,5 | 45,7 |

— $S_1$ = 0,28 m²        $S_2$ = 0,41 m²

| débit ml/min | rdt net % | rdt brut % |
|---|---|---|
| 20 | 43,1 | 34,2 |
| 40 | 50,4 | 46,2 |
| 60 | 45,4 | 45,1 |
| 80 | 46,6 | 39,7 |

— $S_1$ = 0,41 m²        $S_2$ = 0,28 m²

| débit ml/min | rdt net % | rdt brut % |
|---|---|---|
| 20 | 37,45 | 27,4 |
| 40 | 38,7 | 37,7 |
| 60 | 38 | 37,8 |
| 80 | 38 | 37,15 |

Les résultats ainsi obtenus et comparés à ceux de la figure 8 indiquent que selon certaines conditions expérimentales optimales il est possible d'améliorer le rendement d'encapsulation d'une substance dialysable et que ces valeurs sont proches de 45 à 50 % en rendement final, selon le globule rouge utilisé. De même, les valeurs des rendements net et brut se rapprochent, ce qui traduit la diminution de la perte de substance dialysable. Des conditions optimales peuvent être recherchées pour des valeurs différentes de $S_1$ et $S_2$.

## EXEMPLE 13

**Influence de l'âge du globule rouge**

Les essals effectués ont montré qu'il n'y a pas de variation significative du rendement d'encapsulation lorsque les hématies utilisées ont été prélevées depuis moins de 5 jours. Une diminution progressive et lente du rescellement se produit après cette date.

Les exemples suivants donnent les principaux résultats expérimentaux obtenus avec les globules rouges contenant de l'IHP encapsulé par le procédé de l'exemple 5.

## EXEMPLE 14

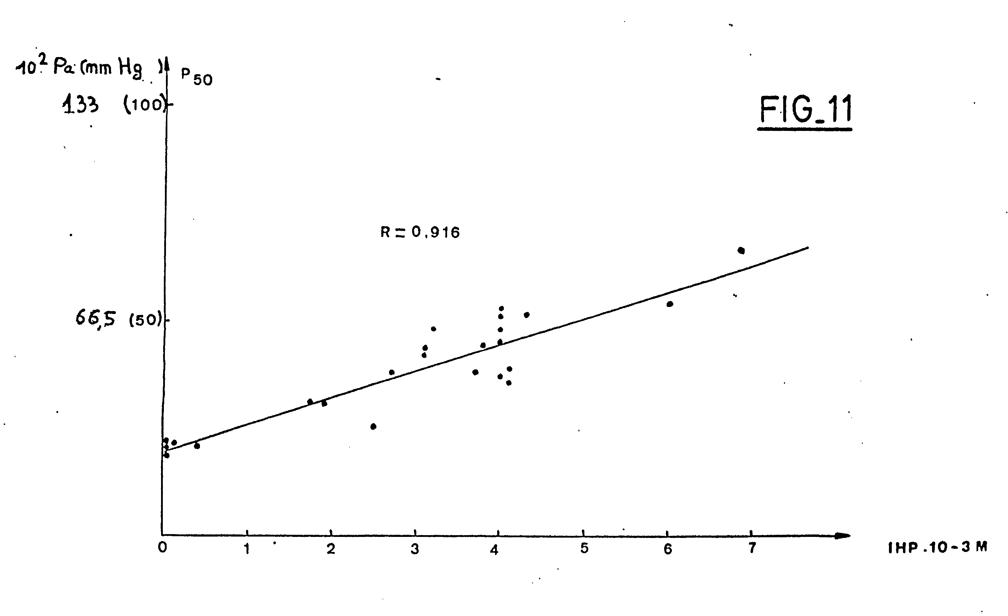

La figure 11 indique la relation obtenue entre la P50 de la courbe de saturation et la concentration en IHP incorporé dans les globules rouges humains. Le coefficient de corrélation est de 0,916.

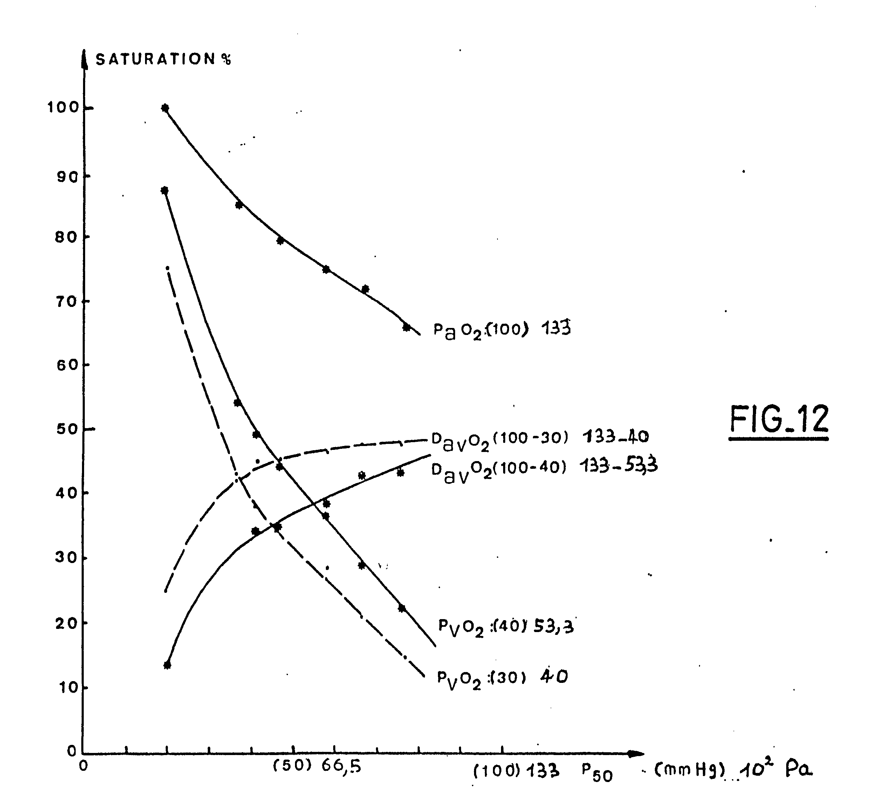

Les courbes de la figure 12 indiquent les modifications de la saturation de l'hémoglobine en fonction de la pression d'oxygène.

L'étude des courbes de la figure 12 indique que malgré une baisse de la saturation de l'hémoglobine pour une pression artérielle de $133.10^2$ Pa (100 mm de Hg), la différence artério-veineuse obtenue à $40.10^2$ et $55.10^2$ Pa(30 et 40 mm de Hg) est très significativement augmentée. Ces différences sont déjà très importantes pour un déplacement de 10 à 20 min des P50 des courbes de saturation.

## EXEMPLE 15

Des globules de porc transformés dans des conditions équivalentes aux globules humains de l'exemple 14 ont été transfusés à des animaux (mini-porc) par exanguino-transfusion. Des volumes de 300 à 500 ml reconstitués à l'hématocrite de l'hématocrite de l'animal ont été utilisés dans chaque essai.

L'équilibre acido-basique et les paramètres de l'oxygénation sont donnés avant et après transfusion dans le tableau ci-dessous:

|  |  | Avant transfusion | Après transfusion |
|---|---|---|---|
| $P_{50}$(mmHg) | Pa | (30.7 $\pm$ 1.0) 4090 $\pm$ 133 | (41.2 $\pm$ 4.0[+++]) 5491 $\pm$ 533 |
| pHa |  | 7.36 $\pm$ 0.05 | 7.34 $\pm$ 0.04 |
| pH$\bar{\text{v}}$ |  | 7.33 $\pm$ 0.05 | 7.28 $\pm$ 0.05 |
| $PaCO_2$(mmHg) | Pa | (39.0 $\pm$ 3.2) 5198 $\pm$ 426 | (35.8 $\pm$ 5.4) 4772 $\pm$ 719 |
| $PvCO_2$(mmHg) | Pa | (48.1 $\pm$ 6.5) 6411 $\pm$ 866 | (45.3 $\pm$ 10.5) 6038 $\pm$ 1399 |
| $CO_2$Ta mm/l |  | 23.3 $\pm$ 3.5 | 19.0 $\pm$ 4.8 |
| $CO_2$T$\bar{\text{v}}$ mm/l |  | 25.9 $\pm$ 3.6 | 21.3 $\pm$ 5.8 |
| Hb g/100 ml |  | 11.1 $\pm$ 2.0 | 11.2 $\pm$ 1.5 |

[+++] $p < 0.001$

Le transport de l'oxygène et la cession tissulaire de l'oxygène sont donnés avant et après transfusion dans le tableau ci-dessous:

|  |  | Avant transfusion | Après transfusion |
|---|---|---|---|
| $PaO_2$(mmHg) | Pa | (82.5 $\pm$ 10,3) 10997 $\pm$ 1373 | (97.5 $\pm$ 10,6) 12996 $\pm$ 14133 [+] |
| $P\bar{\text{v}}O_2$(mmHg) | Pa | (36.7 $\pm$ 5,3) 4892 $\pm$ 706 | (35.1 $\pm$ 3.7) 4678 $\pm$ 493 |
| $CaO_2$ ml/100 ml |  | 13.9 $\pm$ 2.5 | 14.1 $\pm$ 1.5 |
| $C\bar{\text{v}}O_2$ ml/100 ml |  | 9.2 $\pm$ 2.9 | 5.6 $\pm$ 1.7 [+++] |
| DAV ml/100 ml |  | 4.8 $\pm$ 1.1 | 7.6 $\pm$ 0.3 [+++] |
| Q̇ l/mn/kg |  | 0.210 $\pm$ 0.029 | 0.144 $\pm$ 0.054 [+++] |
| $\dot{\text{V}}O_2$ ml/mn/kg |  | 10.3 $\pm$ 1.6 | 10.6 $\pm$ 3.6 |
| P̄art (mmHg) | Pa | (91.5 $\pm$ 10.2) 12196 $\pm$ 1359 | (87.2 $\pm$ 22.8) 11623 $\pm$ 2972 |

[+] $p < 0.05$
[+++] $p < 0.001$

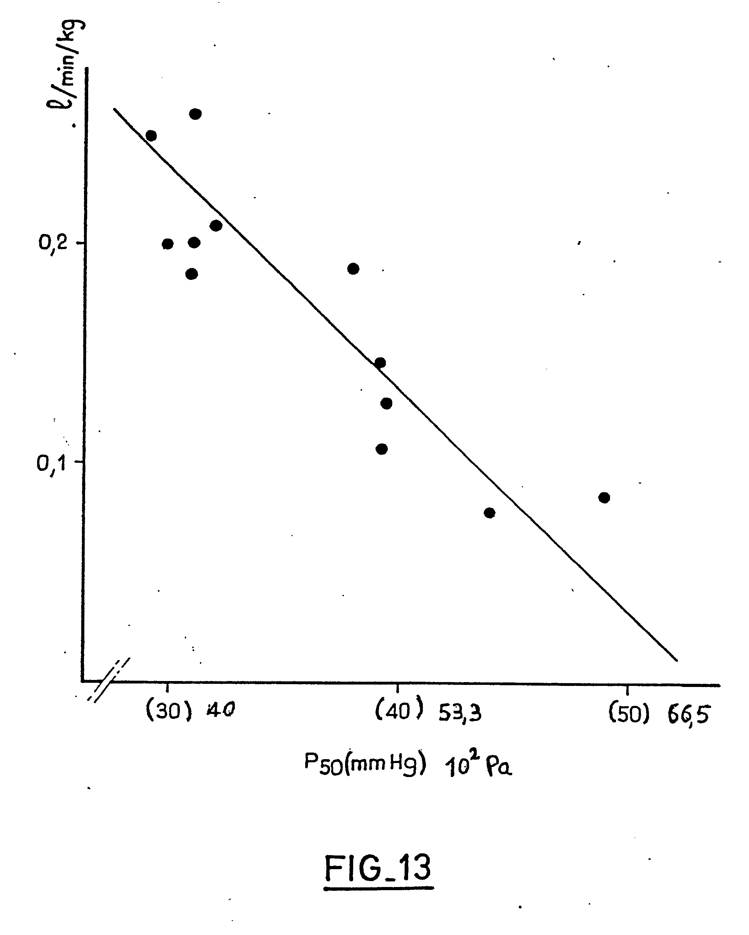

Ces résultats indiquent que, pour une diminution de l'affinité de l'hémoglobine pour l'oxygène, l'animal entier répond par une diminution du débit cardiaque et un élargissement de sa différence artério-veineuse. La cession d'oxygène tissulaire est ainsi favorisée par rapport au transport de l'oxygène. La figure 13 indique l'évolution du débit cardiaque en fonction de la P50 chez six porcs exanguino-transfusés avec des globules de porc ayant incorporé des quantités variables d'IHP.

## EXEMPLE 16

### Caractérisation des hématies rescellées

1°) Microscopie électronique

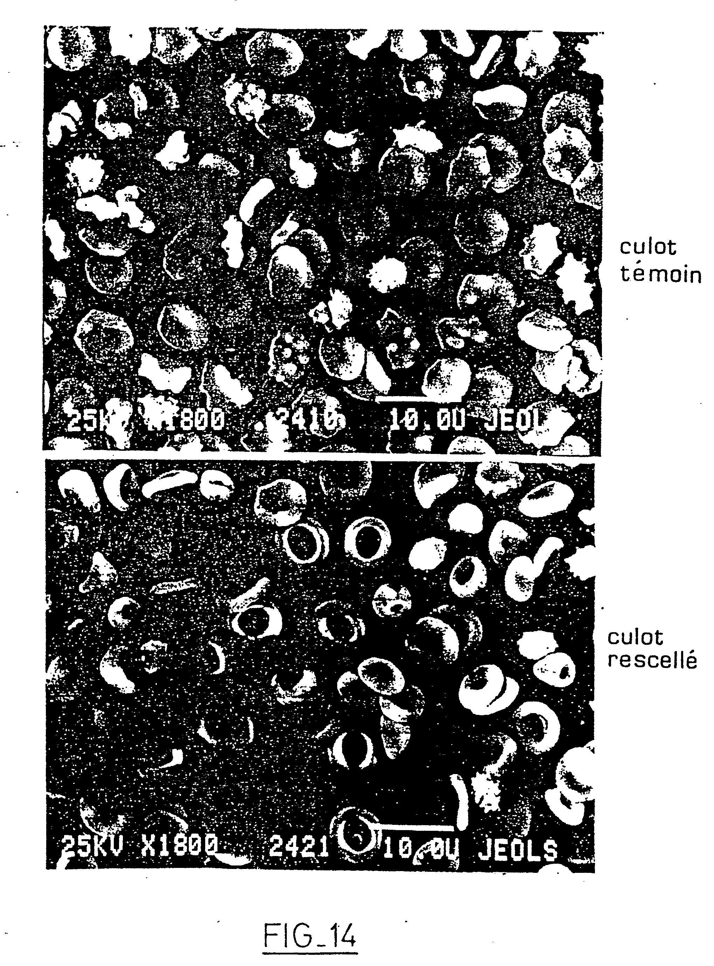

Comme pour la figure 6 avec l'IHP, la figure 14 montre, en microscopie électronique à balayage la morphologie comparée des globules rouges témoins, légèrement échynocytaires, par rapport aux globules lysés et rescellés ayant encapsulé du Desféral. Ces derniers sont de taille comparable. La tendance échynocytaire est réduite. Il s'agit essentiellement de stomatocytes de type I ou II - 5 à 10 % de la population est constituée de formes sphéro-échynocytaires ou de vésicules.

2°) Caractéristiques hématologiques

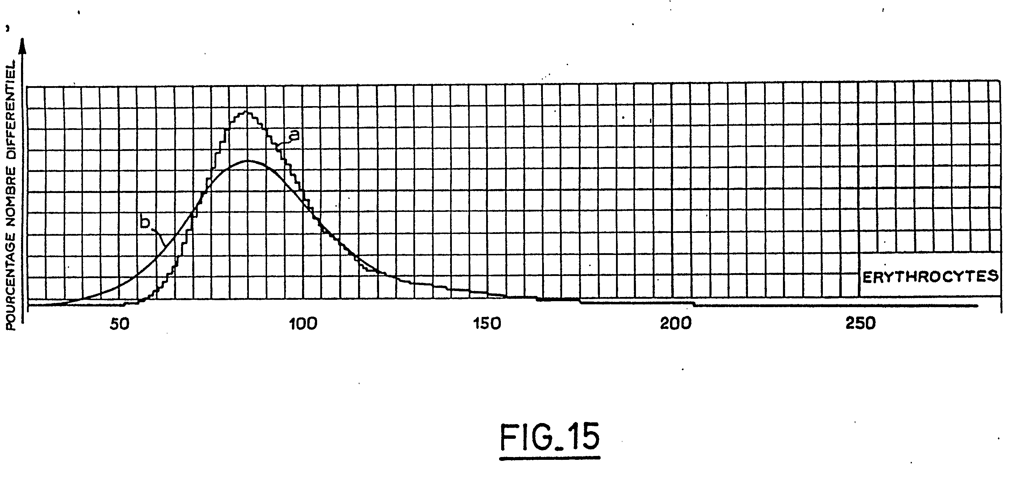

La figure 15 compare la distribution érythrocytaire obtenue au Coulter S d'un culot globulaire lavé (courbe a) à celle qui est obtenue après rescellement (courbe b). Un léger glissement de la distribution vers les formes microcytaires est observé.

Le tableau ci-dessous regroupe les principales caractéristiques de ces globules rouges.

| | Horne (4) | | Porc (2) | |
|---|---|---|---|---|
| | témoin | transformé | témoin | transformé |
| Volume globulaire moyen ($10^{-6}$ $m^3$) | $94,3 \pm 4$ | $84 \pm 3,1$ | 59 | 52,2 |
| Conc. hémoglobine globulaire moyenne (G/100 ml) | $32,3 \pm 1,9$ | $30 \pm 2,5$ | 31 | 32,2 |
| Indice de distribution globulaire | $15,4 \pm 2,8$ | $23,1 \pm 7,2$ | 21,3 | 29,2 |

Il apparaît d'après ces résultats que les hématies lysées et rescellées selon le procédé sont faiblement microcytaires et normochromes, avec un élargissement de la distribution volumique vers les valeurs faibles.

3°) Caractéristiques immunologiques

Les hématies lysées et rescellées ont été examinées pour des modifications qualitatives et quantitatives des antigènes de groupes sanguins. Les méthodes conventionnelles de l'immunohématologie ont été utilisées y compris les tests de Coombs et le traitement aux enzymes protéolytiques.

Les aggrégats obtenus ont une taille un peu réduite mais sont complets et parfaitement visibles. De telles hématies peuvent donc, après leur transformation, être compatibilisées par rapport à un receveur potentiel.

Aucune modification significative, quantitative ou qualitative, n'a été observée, pour des hématies ayant encapsulé du Desféral, dans les systémes antigéniques suivants:

ABO - Rh - CcDEe - Kk kpa kpb - MNSs - $P_1$ - le$^a$ - le$^b$ - Fy$^a$ Fy$^b$ - JK$^a$ - JK$^b$ - lu$^a$.

Ainsi que pour les antigènes: Ku - Gerbich - Fy$^3$ - JK$^3$,

les antigènes du système P - les antigénes Cartwright et Vel.

Aucune anomalie des antigènes de polyagglutinabilité n'est observée en sérum AB.

Ces résultats indiquent clairement la non modification des antigènes publics, privés ou de polyagglutinabilité pour les hématies transformées. Ce résultat est essentiel pour l'utilisation in vivo de ces hématies comme transporteurs de substances d'intérêt biologique en particulier immunomodulateurs susceptibles d'agir sur la réponse immunitaire du receveur.

## EXEMPLE 17

### Encapsulation de MOP et de ses dérivés

Parmi les nombreux immunomodulateurs de synthése ou d'hémi-synthése la famille du muramyldipeptide semble trés prometteuse.

On a vérifié qu'il est possible d'encapsuler certaines de ces substances à l'aide du procédé.

On a étudié la stabilité dans le temps, à l'intérieur des érythrocytes stockés à 4°C.

100 mg de Murabutide® (butyl MDP) (Choay) dans 10 ml de sérum physiologique sont mélangés à 200 ml de culot globulaire lavé, à 70 % d'hématocrite. Après lyse et rescellement selon le procédé de l'exemple 1, 23 mg (23 %) sont retrouvés dans 96 ml de culot globulaire rescellé et lavé, ayant un hématocrite de 64%.

Durant une durée de 8 jours, à l'aide d'un dosage effectué par HPLC, aucune modification significative de la concentration de cette substance dans les érythrocytes conservés ü 4°C n'est observée, ce qui signifie que ce

17

type de composé peptidique n'est pas dégradé à l'intérieur des érythrocytes durant la conservation, à la différence de ce qui est observé avec l'insuline, en l'absence d'inhibiteurs de protéase.

Durant le même laps de temps, aucune concentration significative de ce dérivé du MDP n'est observé ü l'extérieur des globules rouges, ce qui indique qu'il n'existe pas une fuite significative transmembranaire de ce composé. Le rendement d'encapsulation (23 %) est relativement faible, mais peut être attribué au caractère hydrophobe du composé et à son interaction avec la membrane érythrocytaire.

Une dose thérapeutique normale de 5 mg telle qu'elle est estimée actuellement ne nécessiterait qu'environ 20 ml de sang transformé, ce qui indique l'extrême efficacité de ce mode de transport; une telle opération permet de disposer très aisément, pour le même malade, de 5 doses utiles échelonnées dans le temps. En outre, cet exemple n'est pas limitatif de la dose encapsulable (environ 1 g/100 ml pour le Desféral ®.

## EXEMPLE 18

### Encapsulation d'interféron α

D'une manière identique à celle de l'exemple 17, une solution d'interféron α purifié contenant $2,1.10^6$ unites dans 1 ml est mélangée à 220 ml de culot globulaire. Au préalable, compte tenu de la très faible concentration protéique, le circuit de dialyse est incubé durant 30 min par une solution à 1 % d'albumine humaine pour limiter les pertes par absorption non spécifique dans le circuit de dialyse. Après lyse et rescellement, 840 000 unités d'interféron (Rdt = 38 %) sont retrouvées dans les 125 ml de culot globulaire rescellés et lavés. Il est à remarquer que ce rendement est proche de celui qui a été obtenu pour l'albumine (exemple 4).

Dans les limites du titrage biologique effectué, il n'y a pas de diminution nette du titre de l'interféron, durant une période de 8 jours à 4°C, après son encapsulation dans les globules rouges, ce qui indique une excellente conservation in vitro de ce composé après son encapsulation.

Des rendements d'encapsulation équivalents sont obtenus pour l'interféron ∂ et l'interleukine II.

## Revendications

1) Procédé d'encapsulation dans les érythrocytes humains ou animaux d' au moins une substance présentant une activité biologique, caractérisé en ce qu'on alimente en continu avec une suspension aqueuse d'érythrocytes, le compartiment primaire d'au moins un premier élément de dialyse dont le compartiment secondaire contient une solution aqueuse hypotonique par rapport à la dite suspension aquense, d'érythrocytes afin de lyser les érythrocytes, en ce que le lysat d'érythrocytes est alors mis en contact avec ladite substance présentant une activité biologique et en ce que ensuite, afin de resceller la membrane des érythrocytes, on augmente la pression osmotique et/ou oncotique du lysat érythocytraire

2) Procédé selon la revendication 1, caractérisé en ce que 1 on augmente la pression osmotique du lysat d'érythrocytes en le faisant passer dans le circuit primaire d'un second élément de dialyse dont le circuit secondaire contient une solution hypertonique par rapport au lysat et en ce que 1' on recueille en continu le lysat rescellé.

3) Procédé selon la revendication 1, caractérisé en ce que l'on augmente la pression osmotique du lysat en le mélangeant avec une solution hypertonique et/ou hyperoncotique par rapport audit lysat.

4) Procédé selon 1' une des revendications 1 à 3, caractérisé en ce que la lyse est effectuée à une température comprise entre 0 et 10°C.

5) Procédé selon l'une des revendications 1 à 4, caractérisé en ce que le rescellement est effectué à une température comprise entre 20 et 40°C.

6) Procédé selon l'une des revendications 1 à 5, caractérisé en ce que la substance présentant une activité biologique est ajoutée avant ou pendant la lyse.

7) Procédé selon l'une des revendications 1 à 5, caractérisé en ce qu'on alimente en continu le premier élément de dialyse avec une suspension aqueuse d'érythrocytes dont la tonicité est comprise entre 180 et 220 mos.

8) Procédé selon la revendication 7, caractérisé en ce que la tonicité de la suspension aqueuse d'érythrocytes est amenée entre 180 et 220 mos en alimentant en continu le compartiment primaire d'un élément de dialyse supplémentaire, placé avant ledit premier élément, avec une suspension aqueuse d'érythrocytes, le compartiment secondaire dudit premier élément étant alimenté avec une solution aqueuse hypotonique par rapport à la suspension d'érythrocytes afin d'amener la tonicité du compartiment primaire entre 180 et 220 mos.

9) Procédé selon l'une des revendications 7 et 8, caractérisé en ce que la substance à activité biologique est introduite dans la suspension érythrocytaire avant l'entrée dans le premier élément de dialyse et lorsque la tonicité de la suspension est comprise entre 180 et 220 mos.

10) Procédé selon l'une des revendications 1 à 9, caractérisé en ce que la suspension d'érythrocytes circule au moins deux fois dans le compartiment primaire du premier élément de dialyse.

11) Procédé selon l'une des revendications 1 à 10, caractérisé en ce que le lysat d'érythrocytes est conservé avant d'être rescellé.

12) Procédé selon la revendication 11, caractérisé en ce que le lysat d'érythrocytes est conservé par congélation ou par lyophilisation.

13) Procédé selon l'une des revendications 1 à 12, caractérisé en ce que la substance présentant une activité biologique est choisie parmi:
- les protéines,
- les enzymes,
- les hormones,
- les substances à activité pharmacologique,
- les substances modifiant le métabolisme des érythrocytes,
- les effecteurs allostériques de l'hémoglobine,
- les substances protectrices de l'hémoglobine et des érythrocytes.
- les acides nucléiques.

14) Procédé selon la revendication 13, caractérisé en ce que la substance à activité biologique est un effecteur allostérique de l'hémoglobine.

15) Procédé selon la revendication 14, caractérisé en ce qu' il s' agit d' un composé choisi parmi les sucres phosphates, les polyphosphates et les esters phosphatealcools.

16) Procédé selon la revendication 15, caractérisé en ce qu'il s'agit d'un composé choisi parmi l'inositol hexaphosphate, l'inositol pentaphosphate, l'inositol tétraphosphate, l'inositol triphosphate, l'inositol diphosphate et le diphosphatidynilinositol diphosphate, les nucléotides triphosphates, les nucléotides diphosphates, les nucléotides monophosphates et le phosphate de pyridoxal.

17) Procédé selon la revendication 18, caractérisé en ce que le composé utilisé est l'inositol hexaphosphate.

18) Procédé selon la revendication 13, caractérisé en ce que l'on incorpore un composé choisi parmi la desferrioxamine, l'insuline, l'albumine.

19) Procédé selon la revendication 13, caractérisé en ce que la substance présentant une activité biologique est choisie parmi:
- les prostaglandines
- les leucotriènes
- les cytokines
- les immunomodulateurs
- les interférons
- les interleukines
- les enzymes du métabolisme du glucose
- l'anhydrase carbonique
- les enzymes du métabolisme de l'histamine.

20) Procédé selon la revendication 19, caractérisé en ce que la substance est choisie parmi:
- les interférons, et
- l'interleukine II
- le MDP et ses dérivés
- les hexokinases
- la diamino-oxydase et la méthyl-histamine transférase.

21) Procédé selon la revendication 13, caractérisé en ce que la substance à activité biologique est choisie parmi les dérivés du MDP dans lesquels la chaîne peptidique a été mono- ou di-estérifiée sur le groupement carboxylique de l'acide D-glutamique par des chaînes alkyls de 1 à 10 atomes de carbone, et les dérivés du MDP mono- ou di-estérifiés portant en position alpha une chaîne alkyl de 4 à 10 atomes de carbone et en position gamma une chaîne alkyl ayant de 1 à 3 atomes de carbone.

22) Dispositif destiné à l'encapsulation dans les érythrocytes humains ou-animaux d'au moins une substance présentant une-activité biologique, caractérisé en ce qu'il comporte au moins:
- un premier élément de dialyse comportant au moins un compartiment primaire et un compartiment secondaire séparés par une paroi de dialyse,
- un dispositif d'alimentation en continu de la suspension d' érythrocytes dans le compartiment primaire dudit élément de dialyse,
- un moyen permettant d'amener une solution aqueuse dans le compartiment secondaire de l'élément de dialyse,
- un moyen assurant l'introduction de la substance à activité biologique dans le compartiment primaire de l'élément de dialyse,
- un moyen assurant le transfert de la solution effluente du compartiment primaire dans un ensemble de rescellement comportant au moins un moyen pour augmenter la pression osmotique et/ou oncotique de la solution effluente.

23) Dispositif selon la revendication 22, caractérisé en ce que l'ensemble de rescellement est constitué par un second élément de dialyse comportant au moins un compartiment primaire et un compartiment secondaire séparés par une paroi de dialyse, la solution effluente étant amenée dans le compartiment primaire par le moyen de transfert et le moyen pour augmenter la pression osmotique étant constitué par une solution hypertonique par rapport à la solution effluente, ladite solution hypertonique étant contenue dans le compartiment secondaire de l'élément de dialyse.

24) Dispositif selon la revendication 22, caractérisé en ce que l'ensemble de rescellement est constitué par une enceinte comportant un moyen d'amenée d'une solution hypertonique par rapport à la solution effluente.

25) Dispositif selon l'une des revendications 22 à 24, caractérisé en ce que le dispositif comporte entre l'élément de dialyse et l'ensemble de rescellement un moyen de chauffage.

26) Dispositif selon l'une des revendications 22 à 24, caractérisé en ce que l'ensemble de rescellement est muni d'un moyen de chauffage.

27) Dispositif selon l'une des revendications 22 à 26 caractérisé en ce qu'il est muni d'un moyen de controle de la pression osmotique des suspensions erythrocytaires.

28) Dispositif selon l'une des revendications 22 à 27, caractérisé en ce qu'il est muni de moyen de contrôle de la pression des fluides circulant dans le dispositif.

29) Dispositif selon l'une des revendications 22 à 28, caractérisé en ce qu'il comporte un élément de dialyse supplémentaire comportant au moins un compartiment primaire et un compartiment secondaire séparés par une paroi de dialyse, ledit compartiment primaire étant alimenté par le dispositif d'alimentation en continu et l'effluent de ce compartiment primaire alimentant ledit premier élément de dialyse.

30) Dispositif selon la revendication 29, caractérisé en ce qu'il comporte un moyen d'amenée d'une substance à activité biologique dans le circuit entre l'élément de dialyse supplémentaire et ledit premier élément de dialyse.

31) Dispositif selon la revendication 22, caractérisé en ce qu'il comporte un moyen de recyclage de l'effluent de l'ensemble de rescellement dans le premier élément de dialyse.

32) Suspension d'érythrocytes dans laquelle est encapsulée une substance ü activité biologique obtenue par la mise en oeuvre du procédé selon l'une des revendications 1 à 21.

33) Suspension d'érythrocytes selon la revendication 32, caractérisée en ce que la substance à activité biologique est choisie parmi:
- les protéines,
- les enzymes,
- les hormones,
- les substances à activité pharmacologique,
- les substances modifiant le métabolisme des érythrocytes,
- les effecteurs allostériques de l'hémoglobine,
- les substances protectrices de l'hémoglobine et des érythrocytes,
- les acides nucléiques.

**Patentansprüche**

1. Verfahren zum Einkapseln zumindest eines biologisch aktiven Stoffes in Erythrocyten aus Menschen oder Tieren, dadurch gekennzeichnet, daß eine wässerige Suspension von Erythrocyten kontinuierlich dem Primärabteil zumindest eines ersten Dialyseelementes zugeführt wird, dessen Sekundärabteil zwecks Lyse der Erythrocyten eine gegenüber der erwähnten wässerigen Suspension von Erythrocyten hypotone wässerige Lösung enthält, daß dann das Lysat von Erythrocyten mit dem biologisch wirksamen Stoff in Berührung gebracht wird und daß dann zwecks Verschließens der Membran der Erythrocyten der osmotische Druck und/oder oncotische Druck des Erythrocytenlysats erhöht wird.·

2. Verfahren nach Anspruch 1, dadurch gekennzeichnet, daß der osmotische Druck des Erythrocytenlysats dadurch erhöht wird, daß das Erythrocytenlysat in den Primärkreis eines zweiten Dialyselementes eingeführt wird, dessen Sekundärkreis eine gegenüber dem Lysat hypertone Lösung enthält, und daß das wiederverschlossene Lysat kontinuierlich aufgefangen wird.

3. Verfahren ·nach Anspruch 1, dadurch gekennzeichnet, daß der osmotische Druck des Lysats dadurch erhöht wird, daß das Lysat mit einer gegenüber dem erwähnten Lysat hypertonen und/oder hyperoncotischen Lösung vermischt wird.

4. Verfahren nach einem der Ansprüche 1 bis 3, dadurch gekennzeichnet, daß die Lyse bei einer Temperatur zwischen 0 und 10 °C vorgenommen wird.

5. Verfahren nach einem der Ansprüche 1 bis 4, dadurch gekennzeichnet, daß das Wiederverschließen bei einer Temperatur zwischen 20 und 40 °C vorgenommen wird.

6. Verfahren nach einem der Ansprüche 1 bis 5, dadurch gekennzeichnet, daß der biologisch aktive Stoff vor oder während der Lyse zugesetzt wird.

7. Verfahren nach einem der Ansprüche 1 bis 5, dadurch gekennzeichnet, daß das erste Dialyseelement kontinuierlich mit einer wässerigen Erythrocytensuspension beschickt wird, deren Tonizität zwischen 180 und 220 mos liegt.

8. Verfahren nach Anspruch 7, dadurch gekennzeichnet, daß die Tonizität der wässerigen Erythrocytensuspension dadurch auf einen Wert zwischen 180 und 220 mos gebracht wird, daß dem Primärabteil eines vor dem erwähnten ersten Dialyseelement angeordneten zusätzlichen Dialyseelementes kontinuierlich eine wässerige Erythrocytensuspension und dem Sekundärabteil des erwähnten ersten Dialyseelementes eine gegenüber der Erythrocytensuspension hypotone wässerige Lösung zugeführt wird,um die Tonizität im Primärabteil auf einen Wert zwischen 180 und 220 mos zu bringen.

9. Verfahren nach einem der Ansprüche 7 und 8, dadurch gekennzeichnet, daß der biologisch aktive Stoff in die Erythrocytensuspension vor dem Eintritt in das erste Dialyseelement und dann zugeführt wird, wenn die Tonizität der Suspension zwischen 180 und 220 mos liegt.

10. Verfahren nach einem der Ansprüche 1 bis 9, dadurch gekennzeichnet, daß die Erythrocytensuspension im Primärabteil des ersten Dialyseelementes zumindest zweimal umgewälzt wird.

11. Verfahren nach einem der Ansprüche 1 bis 10, dadurch gekennzeichnet, daß das Erythrocytenlysat vor dem Wiederverschließen konserviert wird.

12. Verfahren nach Anspruch 11, dadurch gekennzeichnet, daß das Erythrocytenlysat durch Einfrieren oder Gefriertrocknen konserviert wird.

13. Verfahren nach einem der Ansprüche 1 bis 12, dadurch gekennzeichnet, daß der biologisch aktive Stoff aus
   - Proteinen,
   - Enzymen,
   - Hormonen,
   - pharmakologisch wirksamen Stoffen,
   - den Erythrocytenstoffwechsel modifizierenden Stoffen,
   - allosterischen Haemoglobin-Effektoren,
   - Schutzstoffen für Haemoglobin und Erythrocyten und
   - Nucleinsäuren ausgewählt wird.

14. Verfahren nach Anspruch 13, dadurch gekennzeichnet, daß der biologisch wirksame Stoff ein allosterischer Haemoglobin-Effektor ist.

15. Verfahren nach Anspruch 14, dadurch gekennzeichnet, daß es sich um eine Verbindung handelt, die aus Zuckerphosphaten, Polyphosphaten und Phosphatalkoholestern ausgewählt ist.

16. Verfahren nach Anspruch 15, dadurch gekennzeichnet, daß es sich um eine Verbindung handelt, die aus Inosithexaphosphat, Inositpentaphosphat, Inosittetraphosphat, Inosittriphosphat, Inositdiphosphat und Diphosphatidynilinositdiphosphat , aus Nucleotidtriphosphaten, Nucleotiddiphosphaten, Nucleotidmonophosphaten und Pyridoxalphosphat ausgewählt ist.

17. Verfahren nach Anspruch 16, dadurch gekennzeichnet, daß die verwendete Verbindung Inosithexaphosphat ist.

18. Verfahren nach Anspruch 13, dadurch gekennzeichnet, daß eine aus Desferrioxamin, Insulin und Albumin ausgewählte Verbindung eingebaut wird.

19. Verfahren nach Anspruch 13, dadurch gekennzeichnet, daß der biologisch aktive Stoff aus
   - Prostaglandinen,
   - Leukotrienen,
   - Zytokinen,
   - Immunomodulatoren,
   - Interferonen,
   - Interleukinen,
   - Enzymen für den Glucosemetabolismus,
   - Kohlensäureanhydrase und
   - Enzymen für den Histaminmetabolismus ausgewählt wird.

20. Verfahren nach Anspruch 19, dadurch gekennzeichnet, daß der Stoff aus
   - Interferonen und
   - Interleukin II,
   - Muramyldipeptid (MDP) und seinen Derivaten,
   - Hexokinasen,
   - Diamino-oxydase und Methyl-histamintransferase ausgewählt wird.

21. Verfahren nach Anspruch 13, dadurch gekennzeichnet, daß der biologisch wirksame Stoff aus MDP-Derivaten, in welchen die Peptidkette an der Carboxylgruppe der D-Glutaminsäure durch Alkylketten mit 1 bis 10 Kohlenstoffatomen mono- oder diverestert ist, und aus mono- oder diveresterten MDP-Derivaten, welche in α-Stellung eine Alkylkette mit 4 bis 10 Kohlenstoffatomen und in γ -Stellung eine Alkylkette mit 1 bis 3 Kohlenstoffatomen tragen, ausgewählt wird.

22. Vorrichtung zum Einkapseln zumindest eines biologisch aktiven Stoffes in Erythrocyten aus Menschen oder Tieren, dadurch gekennzeichnet, daß sie zumindest enthält:
   - ein zumindest ein Primärabteil und ein durch eine Dialysierwandung abgetrenntes Sekundärabteil besitzendes erstes Dialyseelement,
   - eine Zufuhreinrichtung zum kontinuierlichen Zuführen einer Erythrocytensuspension in das Primärabteil des erwähnten Dialyseelementes,
   - eine Einrichtung zum Zuführen einer wässerigen Lösung in das Sekundärabteil des Dialyseelementes,
   - eine Einrichtung zum Zuführen des biologisch aktiven Stoffes in das Primärabteil des Dialyseelementes und
   - eine Einrichtung zum Überführen der aus dem Primärabteil abfließenden Lösung in eine Wiederverschließeinheit aufweist, welche zumindest ein Mittel zum Erhöhen des osmotischen Druckes und/oder oncotischen Druckes der abfließenden Lösung besitzt.

23. Vorrichtung nach Anspruch 22, dadurch gekennzeichnet, daß die Wiederverschließeinheit von einem zumindest ein Primärabteil und ein durch eine Dialysierwandung getrenntes Sekundärabteil besitzenden zweiten Dialyseelement gebildet ist, wobei die abfließende Lösung durch die Überführeinrichtung dem Primärabteil zugeführt wird und das Mittel zum Erhöhen des osmotischen Druckes von einer gegenüber der abfließenden Lösung hypertonen Lösung gebildet ist, die im Sekundärabteil des Dialyseelementes enthalten ist.

24. Vorrichtung nach Anspruch 22, dadurch gekennzeichnet, daß die Wiederverschließeinheit von einem Hohlraum gebildet ist welcher eine Zufuhreinrichtung für eine gegenüber der abfließenden Lösung hypertone Lösung aufweist.

25. Vorrichtung nach einem der Ansprüche 22 bis· 24, dadurch gekennzeichnet, daß diese Vorrichtung zwischen dem Dialyseelement und der Wiederverschließeinheit eine Heizeinrichtung aufweist.

26. Vorrichtung nach einem der Ansprüche 22 bis 24, dadurch gekennzeichnet, daß die Wiederverschließeinheit mit einer Heizeinrichtung ausgestattet ist.

27. Vorrichtung nach einem der Ansprüche 22 bis 26, dadurch gekennzeichnet, daß sie mit einer Regeleinrichtung zum Regeln des osmotischen Druckes der Erythrocytensuspensionen ausgestattet ist.

28. Vorrichtung nach einem der Ansprüche 22 bis 27, dadurch gekennzeichnet, daß sie mit einer Regeleinrichtung zum Regeln des Druckes von in der Vorrichtung strömenden Flüssigkeiten ausgestattet ist.

29. Vorrichtung nach einem der Ansprüche 22 bis 28, dadurch gekennzeichnet, daß sie ein zusätzliches, zumindest ein Primärabteil und ein durch eine Dialysierwandung abgetrenntes Sekundärabteil aufweisendes Dialysierelement aufweist, dessen Primärabteil durch die kontinuierlich arbeitende Zufuhreinrichtung versorgt wird, wobei das Abfließende aus diesem Primärabteil das erwähnte erste Dialyseelement versorgt.

30. Vorrichtung nach Anspruch 29, dadurch gekennzeichnet, daß sie im Strömungsweg zwischen dem zusätzlichen Dialyseelement und dem erwähnten ersten Dialyseelement eine Zufuhreinrichtung für einen biologisch aktiven Stoff aufweist.

31. Vorrichtung nach Anspruch 22, dadurch gekennzeichnet, daß sie eine Rückführeinrichtung zum Zurückführen des aus der Wieder verschließeinheit Abfließenden in das erste Dialyseelement aufweist.

32. Erythrocytensuspension, in welcher ein biologisch aktiver Stoff eingekapselt ist und welche beim Arbeiten nach dem Verfahren gemäß einem der Ansprüche 1 bis 21 erhaltenworden ist.

33. Erythrocytensuspension nach Anspruch 32, dadurch gekennzeichnet, daß der biologisch aktive Stoff aus
- Proteinen,
- Enzymen,
- Hormonen,
- pharmakologisch wirksamen Stoffen,
- den Erythrocytenmetabolismus modifizierenden Stoffen,
- allosterischen Haemoglobineffektoren,
- Schutzstoffen für Haemoglobin und Erythrocyten und
- Nucleinsäuren
ausgewählt ist.


**Claims**

1. Method for the encapsulation in human or animal erythrocytes of at least one substance having a biological activity, characterised in that an aqueous suspension of erythrocytes is supplied continuously to the primary compartment of at least one first dialysis member, the secondary compartment of which contains a hypotonic aqueous solution with respect to the said aqueous suspension of erythrocytes in order to bring about the lysis of the erythrocytes, in that the lysate of erythrocytes is thus brought into contact with the said substance having a biological activity and in that then, in order to re-seal the membrane of the erythrocytes, the osmotic and/or oncotic pressure of the erythrocytic lysate is increased.

2. Method according to Claim 1, characterised in that the osmotic pressure of the lysate of erythrocytes is increased by causing it to pass into the primary circuit of a second dialysis member whereof the secondary circuit contains a hypertonic solution with respect to the lysate and in that the re-sealed lysate is recovered continuously.

3. Method according to Claim 1, characterised in that the osmotic pressure of the lysate is increased by mixing it with a hypertonic and/or hyperoncotic solution with respect to the said lysate.

4. Method according to one of Claims 1 to 3, characterised in that the lysis is carried out at a temperature comprised between 0 and 10°C.

5. Method according to one of Claims 1 to 4, characterised in that re-sealing is effected at a temperature of between 20 and 40°C.

6. Method according to one of Claims 1 to 5, characterised in that the substance having a biological activity is added before or during the lysis.

7. Method according to one of Claims 1 to 5, characterised in that the first dialysis member is supplied continuously with an aqueous suspension of erythrocytes whereof the tonicity is comprised between 180 and 220 mos.

8. Method according to Claim 7, characterised in that the tonicity of the aqueous suspension of erythrocytes is brought to between 180 and 220 mos by continuously supplying the primary compartment of an additional dialysis member, located in front of the said first member, with an aqueous suspension of erythrocytes, the secondary compartment of the said first member being supplied with a hypotonic aqueous solution with respect to the suspension of erythrocytes in order to bring the tonicity of the primary compartment to between 180 and 220 mos.

9. Method according to one of Claims 7 and 8, characterised in that the substance having biological activity is introduced into the erythrocytic suspension before entering the first dialysis member and when the tonicity of the suspension is comprised between 180 and 220 mos.

10. Method according to one of Claims 1 to 9, characterised in that the suspension of erythrocytes circulates at least twice in the primary compartment of the first dialysis member.

11. Method according to one of Claims 1 to 10, characterised in that the lysate of erythrocytes is preserved before being re-sealed.

12. Method according to Claim 11, characterised in that the lysate of erythrocytes is preserved by freezing or by lyophilisation.

13. Method according to one of Claims 1 to 12, characterised in that the substance having a biological activity is chosen from:
- proteins,
- enzymes,
- hormones,
- substances having a pharmacological activity,
- substances modifying the metabolism of the erythrocytes,
- allosteric effectors of haemoglobin
- protective substances of haemoglobin and erythrocytes,
- nucleic acids

14. Method according to Claim 13, characterised in that the substance having a biological activity is an allosteric effector of haemoglobin.

15. Method according to Claim 14, characterised in that it is a compound chosen from phosphate sugars, polyphosphates and phosphate-alcohol esters.

16. Method according to Claim 15, characterised in that it is a compound chosen from hexaphosphate inositol, pentaphosphate inositol, tetraphosphate inositol, triphosphate inositol, diphosphate inositol and diphosphate, diphosphatidynilinositol, triphosphate nucleotides, diphosphate nucleotides, monophosphate nucleotides and pyridoxal phosphate.

17. Method according to Claim 16, characterised in that the compound used is hexaphosphate isonitol.

18. Method according to Claim 13, characterised in that a compound chosen from desferrioxamine, insulin, albumin is incorporated.

19. Method according to Claim 13, characterised in that the substance having a biological activity is chosen from:
- prostaglandins
- leucotrienes
- cytokins
- immunomodulators
- interferons
- interleukins
- enzymes of the metabolism of glucose
- carbonic anhydrase
- enzymes of the metabolism of histamine

20. Method according to Claim 19, characterised in that the substance is chosen from:
- interferons and
- interleukin 2
- MDP and its derivatives
- hexokinases
- diamino-oxydase and methyl-histamine transferase

21. Method according to Claim 13, characterised in that the substance having a biological activity is chosen from the derivatives of MDP in which the peptide chain has been mono- or di-esterified on the carboxyl group of D-glutamic acid by alkyl chains having 1 to 10 carbon atoms and the mono- or di-esterified derivatives of MDP comprising an alkyl chain of 4 to 10 carbon atoms at the alpha position and an alkyl chain having from 1 to 3 carbons at the gamma position.

22. Apparatus intended for the encapsulation in human or animal erthyrocytes of at least one substance having a biological activity, characterised in that it comprises at least:
- a first dialysis member comprising at least one primary compartment and one secondary compartment separated by a dialysis wall,
- an arrangement for the continuous supply of the suspension of erythrocytes to the primary compartment of the said dialysis member,
- means making it possible to supply an aqueous solution to the secondary compartment of the dialysis member,
- means ensuring the introduction of the substance having a biological activity into the primary compartment of the dialysis member.

- means ensuring the transfer of the effluent solution from the primary compartment to a re-sealing arrangement comprising at least one means for increasing the osmotic and/or oncotic pressure of the effluent solution.

23. Apparatus according to Claim 22, characterised in that the re-sealing arrangement is constituted by a second dialysis member comprising at least one primary compartment and one secondary compartment separated by a dialysis wall, the effluent solution being supplied to the primary compartment by the transfer means and the means for increasing the osmotic pressure being constituted by a hypertonic solution with respect to the effluent solution, the said hypertonic solution being contained in the secondary compartment of the dialysis member.

24. Apparatus according to Claim 22, characterised in that the re-sealing arrangement is constituted by a chamber comprising means for supplying a hypertonic solution with respect to the effluent solution.

25. Apparatus according to one of Claims 22 to 24, characterised in that the apparatus comprises heating means between the dialysis member and the re-sealing arrangement.

26. Apparatus according to one of Claims 22 to 24, characterised in that the re-sealing arrangement is provided with heating means.

27. Apparatus according to one of Claims 22 to 26, characterised in that means are provided for controlling the osmotic pressure of the erythrocytic suspensions.

28. Apparatus according to one of Claims 22 to 27, characterised in that means are provided for controlling the pressure of the fluids circulating in the apparatus.

29. Apparatus according to one of Claims 22 to 28, characterised in that it comprises an additional dialysis member comprising at least one primary compartment and one secondary compartment separated by a dialysis wall, the said primary compartment being supplied by the continuous supply device and the effluent of this primary compartment supplying the said first dialysis member.

30. Apparatus according to Claim 29, characterised in that it comprises means for supplying a substance having a biological activity in the circuit between the additional dialysis member and the said first dialysis member.

31. Apparatus according to Claim 22, characterised in that. it comprises means for re-cycling the effluent of the re-sealing arrangement into the first dialysis member.

32. Suspension of erythrocytes in which a substance having a biological activity obtained by carrying out the method according to one of Claims 1 to 21 is encapsulated.

33. Suspension of erythrocytes according to Claim 32, characterised in that the substance having a biological activity is chosen from:
- proteins,
- enzymes,
- hormones,
- substances having a pharmacological activity,
- substances modifying the metabolism of erythrocytes,
- allosteric effectors of haemoglobin,
- substances protecting haemoglobin and erythrocytes,
- nucleic acids.

FIG.1

FIG_2

FIG_3

## FIG. 4

SATURATION %

pH 7,4
$PCO_2 = (40\,mm\,Hg)$ $53,3 \cdot 10^2\,Pa$
$T = 37°C$

1  T  2  3  4  5  6  7

100

50

0

(50)  (100)  (150)  (200)
66,6  133   200   266

0   $PO_2(mm\,Hg)$
    $10^2 \cdot Pa$

FIG_5

FIG_6

hémolyse %

100

50

solution de prélavage (mos)

1 gl. 130 NaCl 130

2 gl. 160 NaCl 100

3 gl. 210 NaCl 50

T NaCl 300

T        1  2  3

0        100        200        300

(mos)

FIG_7

FIG.8

Desféral encapsulé
(g/100 ml culot rescellé)

FIG.9

dose de Desféral
(g/200ml culot globulaire)

0 101 341

hémolyse %

INFLUENCE DE LA TEMPERATURE
DE RESCELLEMENT (°C)

45  42  40    35   30        T

42        45       40      30   35

100         200         300 (mos)

FIG_10

19

FIG_11

FIG.12

FIG_13

culot
témoin

culot
rescellé

FIG_14

FIG.15

ERYTHROCYTES

0 101 341